(19) Europäisches Patentamt / European Patent Office / Office européen des brevets

(11) **EP 4 206 317 A1**

(12) **EUROPEAN PATENT APPLICATION**
published in accordance with Art. 153(4) EPC

(43) Date of publication:
**05.07.2023 Bulletin 2023/27**

(21) Application number: **21861934.4**

(22) Date of filing: **09.08.2021**

(51) International Patent Classification (IPC):
*C12N 1/20* (2006.01)    *A61K 35/744* (2015.01)
*A61P 35/00* (2006.01)    *A23L 33/135* (2016.01)
*C12P 7/56* (2006.01)    *A61K 35/74* (2015.01)
*C12R 1/46* (2006.01)    *A61K 39/00* (2006.01)

(52) Cooperative Patent Classification (CPC):
**A23L 33/135; A61K 35/74; A61K 35/744;**
**A61K 39/00; A61P 35/00; C12N 1/20; C12P 7/56;**
C12R 2001/46

(86) International application number:
**PCT/KR2021/010525**

(87) International publication number:
**WO 2022/045637 (03.03.2022 Gazette 2022/09)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **28.08.2020  KR 20200109411**
        **23.09.2020  KR 20200123330**

(71) Applicant: **LIVEOME Inc.**
**Yeongtong-gu, Suwon-si**
**Gyeonggi-do 16506 (KR)**

(72) Inventors:
• **KIM, Yeung Hyen**
  **Hwaseong-si, Gyeonggi-do 18423 (KR)**

• **KWON, Do Hyeong**
  **Suwon-si, Gyeonggi-do 16509 (KR)**
• **PARK, Kwang Seo**
  **Suwon-si, Gyeonggi-do 16509 (KR)**
• **DONG, Hye Jin**
  **Incheon 21994 (KR)**
• **JANG, Hye Jeong**
  **Seoul 06626 (KR)**
• **SONG, Ji Yoon**
  **Seongnam-si, Gyeonggi-do 13479 (KR)**

(74) Representative: **Brevalex**
**56, Boulevard de l'Embouchure**
**B.P. 27519**
**31075 Toulouse Cedex 2 (FR)**

Remarks:
The complete document including Reference
Table(s) and the Sequence Listing(s) can be
downloaded from the EPO website

(54) **ANTITUMOR BACTERIAL STRAIN, AND COMPOSITION AND METHOD USING SAME**

(57)    Provided are *Enterococcus faecium* microorganisms or *Enterococcus faecalis* microorganisms having anti-cancer activity, or a composition containing the same, and a method of preventing or treating cancer by using the composition.

# FIG. 1

ENTEROCOCCUS FAECIUM
LMT17-62

ENTEROCOCCUS FAECIUM
KCTC13225

**Description**

TECHNICAL FIELD

The present disclosure relates to anti-tumor bacterial strains, and compositions and methods using the same, more specifically, bacterial strains of a species of *Enterococcus faecium* exhibiting anti-tumor activity, and compositions and methods for preventing or treating tumors by using the same.

In addition, the present disclosure relates to anti-tumor bacterial strains, and compositions and methods using the same, and more specifically, to *Enterococcus faecalis* microorganisms having anti-cancer activity, a composition containing the same, and a method of preventing or treating cancer by using the same.

BACKGROUND ART

It is known that gut bacteria may be used to prevent or treat various diseases or disorders.

International Publication No. WO2016/196605 discloses a method of treating or preventing cancer in a subject, by modulating a level of one or more commensal microbes in the subject to: enhance an immune response by the subject; and/or inhibit growth or spread of cancer; and/or inhibit immune evasion of cancer; and/or enhance efficacy of a therapeutic agent. This reference cites genera *Adlercreutzia, Oscillopira, Mollicutes, Butyrivibrio, Bacteroides, Clostridium, Fusobacterium, Eubacterium, Ruminococcus, Peptococcus, Peptostreptococcus, Bifidobacterium, Rikenella, Alistipes, Marinilabilia, Anaerostipes, Escherichia, Lactobacillus* as examples of commensal microbes, and discloses a method of treating cancer by using bacteria of a genus *Bifidobacterium* in combination with an immune checkpoint inhibitor.

International Publication No. WO2017/085520 discloses a composition for use in a method for treating or preventing cancer, including a bacterial strain of a species *Enterococcus gallinarum.*

International Publication No. WO2017/085518 discloses a composition for use in a method of treating or preventing a disease or condition mediated by the IL-17 or Th17 pathway, including a bacterial strain of a species *Enterococcus faecium.*

To date, correlation between anti-tumor activity and an ability to produce a specific metabolite, compared to microbial growth in *Enterococcus faecium* species has not been known.

In addition, *Enterococcus faecalis* is found in gastrointestinal tracts of humans and other mammals. *Enterococcus faecalis* is a gram-positive, cocci bacterium. *Enterococcus faecalis* can grow at a temperature of 10 °C to 45 °C.

Korean Patent Publication No. 1 0-2020-0054589 discloses *Enterococcus faecalis* KACC 92220P having an effect of lowering a lactose content. In addition, Korean Patent No. 1 0-2053730 discloses an *Enterococcus faecalis* AMI-1001 strain having anti-oxidant, anti-inflammatory, or anti-bacterial activity.

However, even according to the above-described related art, there is a demand for *Enterococcus faecalis* having anti-cancer activity.

DESCRIPTION OF EMBODIMENTS

TECHNICAL PROBLEM

A first object of the present disclosure is to provide a bacterial strain of a species *Enterococcus faecium* that has anti-tumor activity, which is characterized by having a lactate production ability compared to microbial growth.

A second object of the present disclosure is to provide a composition for preventing or treating tumors, including the bacterial strain of the *Enterococcus faecium* species as an active ingredient.

A third object of the present disclosure is to provide a method of preventing or treating tumors in a subject, including administering the bacterial strain of the *Enterococcus faecium* species or the composition to the subject.

A fourth object of the present disclosure is to provide *Enterococcus faecalis* LMT19-32 (accession number KCTC 14306BP) microorganisms, which have anti-tumor activity, or a culture or an extract thereof.

A fifth object of the present disclosure is to provide a pharmaceutical composition for preventing or treating cancer containing the *Enterococcus faecalis* LMT19-32 microorganisms or a culture or an extract thereof as an active ingredient.

A sixth object of the present disclosure is to provide a food composition for preventing or ameliorating cancer containing the *Enterococcus faecalis* LMT19-32 microorganisms or a culture or an extract thereof as an active ingredient.

A seventh object of the present disclosure is to provide a method of preventing or treating cancer in a subject including administering to a subject an effective amount of the *Enterococcus faecalis* LMT19-32 microorganisms or a culture or extract thereof for treating cancer.

SOLUTION TO PROBLEM

A first aspect of the disclosure provides a bacterial strain that

i) belongs to a species *Enterococcus faecium,*
ii) has a lactate production ability compared to microbial growth (lactate/OD$_{600}$) of 3 g/L or more, when cultured for 48 hours, and
iii) exhibits anti-tumor activity.

A second aspect of the present disclosure provides a composition for preventing or treating tumor, including the bacterial strain of the *Enterococcus faecium* species as an active ingredient.
A third aspect of the present disclosure is to provide a method of preventing or treating cancer in a subject, including administering to a subject the bacterial strain of the species of *Enterococcus faecalis,* or the composition.
A fourth aspect of the present disclosure is to provide *Enterococcus faecalis* LMT19-32 (accession number KCTC 14306BP) microorganisms, which have anti-tumor activity, or a culture or an extract thereof.
A fifth aspect of the present disclosure is to provide a pharmaceutical composition for preventing or treating cancer, containing the *Enterococcus faecalis* LMT19-32 microorganisms or a culture or an extract thereof as an active ingredient.
A sixth aspect of the present disclosure is to provide a food composition for preventing or ameliorating cancer, containing the *Enterococcus faecalis* LMT19-32 microorganisms or a culture or an extract thereof as an active ingredient.
A seventh aspect of the present disclosure is to provide a method of preventing or treating cancer in a subject including administering to a subject an effective amount of the *Enterococcus faecalis* LMT19-32 microorganisms or a culture or extract thereof for treating cancer.

ADVANTAGEOUS EFFECTS OF DISCLOSURE

The bacterial strain according to the present disclosure increases numbers of tumor-infiltrating T cells, CD8 T cells, and IFN$\gamma^{+}$ CD8 T cells, and thus exhibits excellent tumor suppression activity, and therefore may be effectively used to prevent or treat tumors.
In another aspect, according to the *Enterococcus faecalis* LMT19-32 (accession number KCTC 14306BP) microorganisms having anti-tumor activity or a culture or extract thereof according to the present disclosure, cancer may be prevented or treated.
In another aspect, according to the pharmaceutical composition for preventing or treating cancer according to the present disclosure, cancer may be prevented or treated.
In another aspect, according to the food composition for preventing or ameliorating cancer according to the present disclosure, caner may be prevented or ameliorated.
In another aspect, according to the method of preventing or treating cancer in a subject according to the present disclosure, cancer may be efficiently prevented or treated.

BRIEF DESCRIPTION OF DRAWINGS

FIG. 1 shows optical microscope images of a strain *Enterococcus faecium* LMT17-62 and a type strain KCTC13225 according to an embodiment of the present disclosure.
FIG. 2A is a graph showing tumor growth inhibition rates (%) of *Enterococcus faecium* strains selected from mouse tumor induction models.
FIG. 2B are graphs showing a change in a tumor size over time in a group administered with the strain *Enterococcus faecium* LMT17-62.
FIG. 2C are graphs showing changes in numbers of total tumor-infiltrating T cells, CD8 T cells, and interferon gamma-positive CD8 T cells in the group administered with the strain *Enterococcus faecium* LMT17-62.
FIG. 3A is a growth (OD$_{600}$) curve of a selected *Enterococcus faecium* strain.
FIG. 3B is a graph showing an amount of lactate produced according to incubation time of the selected *Enterococcus faecium* strain.
FIG. 3C are graphs showing amounts of lactate production (g/L) as compared to growth (OD$_{600}$) of selected *Enterococcus faecium* strains after culturing for 24 hours and 48 hours.
FIG. 4 is a graph showing numbers of live bacteria of the selected *Enterococcus faecium* strains when the strains are cultured at pH 2.5.

FIG. 5 is a graph showing numbers of live bacteria of the selected *Enterococcus faecium* strains when the strains are cultured in a bile salt-containing medium.

FIG. 6 is a graph showing numbers of live bacteria of the selected anti-tumor-positive *Enterococcus faecium* strains attached to intestinal epithelial cells.

FIG. 7 shows representative optical microscope images of a selected strain *Enterococcus faecalis* LMT19-32 and a type strain KCTC3206.

FIGS. 8A and 8B are diagrams showing results of measuring tumor sizes after administering the *Enterococcus faecalis* LMT19-32 strain to mice with tumor.

FIGS. 9A, 9B, and 9C show results of analyzing tumor-infiltrating T cells, CD8 T cells, and IFN$\gamma^+$ CD8 T cells after administration of the *Enterococcus faecalis* LMT19-32 strain to mice with tumor.

BEST MODE

*1. Enterococcus faecium*

The present disclosure is based on a finding that bacterial strains of the species *Enterococcus faecium,* which are characterized by their ability to produce lactate compared to microbial growth, exhibit excellent anti-tumor activity.

The terms "tumor" and "cancer" are used interchangeably and encompass solid and liquid, for example, diffuse or circulating tumors. The terms include precancer as well as malignant cancers and tumors. The terms also include primary malignant cells or tumors and secondary malignant cells or tumors (for example, metastatic tumors).

The terms "anti-tumor" and "anti-cancer" may refer to biological effects that may be expressed by a variety of means, including but not limited to, for example, reduction in tumor size, reduction in a number of tumor cells, reduction in tumor cell proliferation, or reduction in tumor cell survival.

An aspect of the disclosure provides a bacterial strain that

i) belongs to a species *Enterococcus faecium,*
ii) has a lactate production ability compared to microbial growth (lactate/$OD_{600}$) of 3 g/L or more, when cultured for 48 hours, and
iii) exhibits anti-tumor activity.

In an embodiment, the bacterial strain may have a lactate production ability (lactate/$OD_{600}$) compared to microbial growth of greater than 2.5 g/L, when the bacterial strain is cultured for 24 hours.

In an embodiment, the bacterial strain may exhibit a tumor growth inhibition rate of 10 % or more, 15 % or more, 20 % or more, 25 % or more, 30 % or more, or 35 % or more. In a certain example, the bacterial strain may exhibit a tumor growth inhibition rate of about 10 % to about 40 %, about 15 % to about 40 %, about 20 % to about 40 %, about 25 % to about 40 %, about 30 % to about 40 %, or about 35 % to about 40 %.

In an embodiment, the bacterial strain may have iv) β-galactosidase activity.

In an embodiment, the bacterial strain may have v) a D-sorbitol decomposition ability.

In an embodiment, the bacterial strain may have vi) a D-tagatose decomposition ability.

In an embodiment, the bacterial strain may have vii) a methyl-αD-mannopyranoside decomposition ability.

In an embodiment, the bacterial strain may satisfy the three characteristics of i) to iii), the four characteristics of i) to iv), five characteristics of i) to v), i) to iv), i) to vi),or i) to vii), six characteristics of i) to vi), or i) to vii), or seven characteristics of i) to vii).

In a specific example, the bacterial strain may be one or more of those shown in Table 1 below, but is not limited thereto.

[Table 1]

| Name | Accession number | Characteristics | | | | |
|---|---|---|---|---|---|---|
| | | Lactate/ $OD_{600}$(g/L) | β-galactosidase | D-sorbitol | D-tagatose | Methyl-αD-mannopyranoside |
| LMT17-62 | KCTC 14284BP | >3 | + | + | + | + |
| LMT17-74 | KCTC 14285BP | >3 | + | + | + | + |

(continued)

| Name | Accession number | Characteristics | | | | |
|---|---|---|---|---|---|---|
| | | Lactate/ $OD_{600}$(g/L) | β-galactosidase | D-sorbitol | D-tagatose | Methyl-α D-mannopy ranoside |
| LMT15-24 | KCTC 14289BP | >3 | + | + | + | - |
| LMT17-25 | KCTC 14288BP | >3 | + | - | - | - |
| LMT15-4 | KCTC 14290BP | >3 | - | - | - | - |

A second aspect of the present disclosure relates to a composition for preventing or treating tumors, comprising the bacterial strain as an active ingredient. The terms "treat," "treating," or "treatment" refer to, for example, healing injured or damaged tissue, achieving desired therapeutic results by altering, changing, strengthening, ameliorating, improving, and/or beautifying a pre-existing or recognized disease, disorder, or condition; or alleviating, reducing (including partial reduction, substantial reduction, near complete reduction, and complete reduction), resolving or preventing (whether temporarily or permanently) of a disease, a disorder, or a condition.

The term "prevention" means delaying an onset of a disease, disorder, or condition. Prevention may be considered complete when an onset of a disease, disorder, or condition is delayed for a pre-determined period of time.

In an embodiment, the bacterial strain included in the composition may exist as live bacteria or dead bacteria, and may exist in a dried or lyophilized form.

In an embodiment, the bacterial strain may be used in any form, such as a culture or an isolated strain, as long as the strain retains its anti-tumor activity, and all forms fall within the scope of the present disclosure. The term "culture" refers to a composition including a cultured strain, its metabolites, and extra nutrients, etc., obtained by culturing the strain for a certain period of time in a medium that can supply nutrients so that the bacterial strain of the present disclosure may grow and survive *in vitro.*

In an embodiment, the composition may be for oral, or parenteral administration. Parenteral administration includes, but is not limited to, intravenous injection, subcutaneous injection, intramuscular injection, intraperitoneal injection, intradermal administration, topical administration, intranasal administration, intrapulmonary administration, intrarectal administration, intratumoral administration, and the like. In a specific example, the composition may be for oral administration.

The composition may be a pharmaceutical composition, in which case the pharmaceutical composition may be formulated by further including a pharmaceutically acceptable carrier or additive, in addition to the active ingredient. The carrier or additive may include an excipient, a disintegrant, a sweetener, a binder, a coating agent, an expanding agent, a lubricant, a glydent, a flavoring agent, a coloring agent, a diluent, a dispersing agent, a surfactant, an antioxidant, a buffer, a bacteriostatic agent, and the like. The composition may be formulated into a pill, a powder, a capsule, a granule, or a tablet, or an injectable formulation such as an aqueous solution, suspension, or emulsion.

The composition may be a food or a food additive composition, in which case the composition may include a sitologically acceptable diluent or carrier. The diluent may be water, medium or a buffer such as PBS. The carrier may be a commonly used excipient, disintegrant, binder, glydent, thickener, or filler. The food may be a health functional food. The food may be a beverage, confectionery, diet bar, chocolate, pizza, ramen, other noodles, chewing gum, ice cream, and the like.

When the composition is for oral administration, it may be coated with a coating agent to increase acid resistance, thermal resistance, bile resistance, survival rate, intestinal anchorage, etc. of the bacterial strain. A coating agent that may be used include, but is not particularly limited to, enteric coating agents; gelatin, polysaccharides, gums and the like; water soluble polymers, hyaluronic acid, porous particles, and proteins; casein, and coating agent, edible fat and oil, extracellular polymeric substance of *Lactobacillus plantarum,* and alginic acid; silk fibroin, and the like, and the coating may be a single coating or multiple coatings, for example, double, triple, or quadruple coatings.

In an embodiment, the tumor may be a solid tumor. Non-limiting examples of the solid tumor include, but are not limited to, breast cancer, lung cancer, head or neck cancer, colorectal cancer, esophageal cancer, laryngeal cancer, stomach cancer, liver cancer, pancreatic cancer, bone cancer, skin cancer, skin or intraocular melanoma, uterine cancer, ovarian cancer, rectal cancer, proximal anal cancer, colon cancer, breast cancer, fallopian tube carcinoma, endometrial carcinoma, cervical carcinoma, vaginal carcinoma, vulvar carcinoma, Hodgkin's disease, small intestine cancer, endocrine cancer, thyroid cancer, parathyroid cancer, adrenal cancer, soft tissue sarcoma, urethral cancer, penile cancer, prostate cancer, lymphocytic lymphoma, bladder cancer, renal or ureteric cancer, renal cell carcinoma, renal pelvic carcinoma, CNS tumor, primary CNS lymphoma, spinal cord tumor, brainstem glioma, and pituitary adenoma.

In an embodiment, a dosage may vary depending on the patient's body weight, age, sex, health condition, diet, excretion rate, and constitutional specificity, administration time administration method, administration period or interval, nature of the formulation, severity of the disease, etc. and may be appropriately selected by a person skilled in the art. For example, the bacterial strain, which is an active ingredient, may be administered in an amount of $1 \times 10^6$ CFU or more, $1 \times 10^7$ CFU or more, $1 \times 10^8$ CFU or more, $1 \times 10^9$ CFU or more. For example, the bacterial strain may be administered in an amount of $1 \times 10^{15}$ CFU or less, $1 \times 10^{14}$ CFU or less, $1 \times 10^{13}$ CFU or less, $1 \times 10^{12}$ CFU or less. For example, the bacterial strain may be administered in an amount of about $1 \times 10^6$ to about $1 \times 10^{15}$ CFU, about $1 \times 10^7$ to about $1 \times 10^{14}$ CFU, about $1 \times 10^8$ to about $1 \times 10^{13}$ CFU, about $1 \times 10^9$ to about $1 \times 10^{12}$ CFU, about $1 \times 10^{10}$ to about $1 \times 10^{12}$ CFU, or about $1 \times 10^{11}$ to about $1 \times 10^{12}$ CFU. For example, the bacterial strain, which is an active ingredient, may be administered once a day or several times a day in aliquots.

In an embodiment, the composition may be for using in combination with one or more other therapeutic agents, for example, anti-cancer agents, anti-viral agents, cytokines, or immuneagents.

The term "use in combination" refers to any form of administration of two or more different therapeutic agents such that a second therapeutic agent is administered while a previously administered therapeutic agent is still effective in the body. For example, two therapeutic agents are simultaneously effective in a subject, and there may be a synergistic effect of the two therapeutic agents. The different therapeutic agents may be administered concurrently or sequentially in a single formulation or in separate formulations.

In a specific example, the anti-cancer agent may be a chemotherapeutic agent. Non-limiting examples of the chemotherapeutic agents include alkylating agents, nitrosoureases, anti-metabolites, anti-cancer anti-biotics, plant-derived alkaloids, topoisomerase inhibitors, hormonal drugs, hormone antagonists, leukopenia (neutropenia) treatment drugs, thrombocytopenia drugs, anti-emetics, aromatase inhibitors, P-glycoprotein inhibitors, platinum complex derivatives, and other immunotherapy drugs and other anti-cancer drugs. Cytotoxic agents that may be coadministered include, for example, anti-microtubule agents, topoisomerase inhibitors, anti-metabolites, mitotic inhibitors, alkylating agents, anthracyclines, vinca alkaloids, intercalating agents, agents that may interfere with signal transduction pathways. agents that promote apoptosis, proteasome inhibitors, and radiation (local or systemic irradiation). Non-limiting examples of additional therapeutic agents include, but are not limited to, peptides, polypeptides, proteins, fusion proteins, nucleic acid molecules, small molecules, mimetic agents, synthetic drugs, inorganic and organic molecules.

In a specific example, the immuno-anti-cancer agent may be a chemotherapeutic agent. The term "immuno-anti-cancer agent" refers to a compound, composition or treatment that indirectly or directly enhances, stimulates or increases the body's immune response to cancer cells and/or reduces side effects of other anti-cancer therapies. Non-limiting examples of immuno-anti-cancer agents include cytokines, cancer vaccines, monoclonal antibodies, non-cytokine adjuvants, immune cells (T cells, NK cells, dendritic cells, B cells, etc.), immune checkpoint inhibitors, and the like. In a specific example, the immuno-anti-cancer agent is an immune checkpoint inhibitor. Immune checkpoint inhibitors include peptides, antibodies, nucleic acid molecules, and small molecules. For example, an immune checkpoint inhibitor may be administered to enhance proliferation, migration, persistency and/or cytotoxic activity of CD8+ T cells in a subject, and in particular, tumor infiltration of CD8+ T cells in a subject. Typically, immune checkpoint inhibitors target activated T lymphocytes, such as cytotoxic T lymphocyte-associated protein 4 (CTLA4) and programmed cell death 1 (PD-1), or various members of the killer cell immunoglobulin-like receptor (KIR) family, which are antagonists that block immunosuppressive receptors expressed by NK cells, or antagonists that block key ligands of the receptor, for example, PD-1 ligand CD274 (best known as PD-L1 or B7-H1). For example, an immune checkpoint inhibitor is an antibody or an antigen-binding fragment thereof. Specifically, an immune checkpoint inhibitor may be at least one selected from the group consisting of anti-PD-1 antibodies, anti-PD-L1 antibodies, anti-PD-L2 antibodies, anti-CTLA-4 antibodies, anti-TIM-3 antibodies, anti-LAG3 antibodies, anti-IDO1 antibodies, anti-TIGIT antibodies, anti-B7H3 antibodies, anti-B7H4 antibodies, anti-BTLA antibodies, and anti-B7H6 antibodies, and antigen-binding fragments thereof. More specifically, an immune checkpoint inhibitor may be at least one selected from ipilimumab (Yervoy®, BMS/Ono), tremelimumab (AstraZeneca), atezolizumab (Tecentriq®, Roche), nivolumab (Opdivo®, BMS/Ono), pembrolizumab (Keytruda®, MSD), avelumab (Bavencio®, Pfizer/Merck, Germany), durvalumab (Imfinzi®, AstraZeneca/Medimmune), and antigen-binding fragments thereof, but is not limited thereto.

In an embodiment, the immuno-anti-cancer agent may be formulated and used in various forms suitable for each purpose of use according to a method commonly used in the art, for example, as an oral formulation such as a liquid, suspension, powder, granule, tablet, capsule, pill, extract, emulsion, syrup, and aerosol, and a parenteral formulation such as an injection of a sterile injection solution. Immuno-anti-cancer agents may be administered orally or parenterally through various routes, including intravenous, intraperitoneal, subcutaneous, intradermal, intramuscular, spinal, intrathecal, rectal local administration, or injection. A dosage may vary depending on the patient's body weight, age, sex, health condition, diet, excretion rate, and constitutional specificity, administration time, administration method, administration period or interval, nature of the formulation, severity of the disease, etc. and may be appropriately selected by a person skilled in the art. For example, the dosage may be in a range of about 0.1 mg/kg to about 10,000 mg/kg, but is not limited thereto, and may be administered once a day or several times a day in aliquots.

A third aspect of the present disclosure provides a method of preventing or treating cancer in a subject, including administering to a subject the bacterial strain, or the composition.

The term "administration" or "administering" means a process of providing an active ingredient or a composition including the same to a subject. The active ingredient or the composition including the same may be administered through various appropriate routes.

Subjects to be administered may include humans or animals, for example, humans, pigs, dogs, cats, cows, horses, mice, and the like without limitation.

## II. *Enterococcus faecalis* LMT19-32

The first aspect of the present disclosure provides *Enterococcus faecalis* LMT19-32 (accession number KCTC 14306BP) microorganisms, which have anti-tumor activity, or a culture or an extract thereof.

The microorganisms, or the culture or the extract thereof may inhibit tumor growth. The microorganisms, or the culture or the extract thereof may increase a level of immune cells in a tumor. The microorganisms, or the culture or the extract thereof may increase a level of CD8 T cells in a tumor, for example, CD8 T cells expressing IFNγ. The microorganisms, or the culture or the extract thereof may increase a level of infiltration of immune cells into a tumor. The immune cells may be CD8 T cells, CD4 T cells, NK cells, B cells, dendritic cells, macrophages, and neutrophils. The microorganisms, or the culture or the extract thereof may activate immune cells. The activation may be such that the microorganisms, or the culture or the extract thereof promotes production, secretion, or production and secretion of cytokines or enzymes having anti-tumor activity. The cytokine or enzyme may be one or more of interferon gamma and granzyme B. The microorganisms are excellent in bile resistance and intestinal anchorage. The microorganisms are isolated from human feces.

When the microorganisms are cultured in De Man, Rogosa and Sharpe agar (MRS) medium containing 0.3 % of bile acid for 2 hours at 37 °C, the bile acid resistance may be 65 % or more, 70 % or more, 75 % or more, 80 % or more, 85 % or more, 90 % or more, 93 % or more, 95 % or more, about 75 % to about 90 %, about 75 % to about 95 %, about 80 % to about 90 %, about 80 % to about 95 %, about 85 % to about 90 %, or about 90 % to about 95 % of survival rate.

The microorganisms, or the culture or the extract thereof may promote infiltration of CD8 T cells into tumor. The promotion may increase a percentage of CD8 T cells to the number of T cells in the tumor by 5 % or more, 10 % or more, 15 % or more, 20 % or more, 25 % or more, 30 % or more, 35 % or more, 45 % or more, 50 % or more, 55 % or more, 65 % or more, 70 % or more, 75 % or more, 80 % or more, 85 % or more, 90 % or more, 100 % or more, about 5 % to about 100 %, about 10 % to about 100 %, about 20 % to about 100 %, about 30 % to about 100 %, about 40 % to about 100 %, about 50 % to about 100 %, about 60 % to about 100 %, about 70 % to about 100 %, about 80 % to about 100 %, or about 90 % to about 100 %, compared to a case in which the microorganisms, or the culture or the extract thereof is not present.

The microorganisms, or the culture or the extract thereof may promote production, secretion, or production and secretion of one or more of interferon gamma and granzyme B in tumor-infiltrating CD8 T cells. The promotion may increase a percentage of cells producing interferon gamma among CD8 T cells by 5 % or more, 10 % or more, 15 % or more, 20 % or more, 25 % or more, 30 % or more, 35 % or more, 45 % or more, 50 % or more, 55 % or more, 65 % or more, 70 % or more, 75 % or more, 80 % or more, 85 % or more, 90 % or more, 100 % or more, about 5 % to about 100 %, about 10 % to about 100 %, about 20 % to about 100 %, about 30 % to about 100 %, about 40 % to about 100 %, about 50 % to about 100 %, about 60 % to about 100 %, about 70 % to about 100 %, about 80 % to about 100 %, or about 90 % to about 100 %, compared to the case in which the microorganisms, or the culture or the extract thereof is not present. In addition, the promotion may increase a percentage of cells producing granzyme B among CD8 T cells by 5 % or more, 10 % or more, 15 % or more, 20 % or more, 25 % or more, 30 % or more, 35 % or more, 45 % or more, 50 % or more, 55 % or more, 65 % or more, 70 % or more, 75 % or more, 80 % or more, 85 % or more, 90 % or more, 100 % or more, about 5 % to about 100 %, about 10 % to about 100 %, about 20 % to about 100 %, about 30 % to about 100 %, about 40 % to about 100 %, about 50 % to about 100 %, about 60 % to about 100 %, about 70 % to about 100 %, about 80 % to about 100 %, or about 90 % to about 100 %, compared to the case in which the microorganisms, or the culture or the extract thereof is not present.

The microorganisms, or the culture or the extract thereof may suppress growth of a tumor, by CD8 T cells. The suppression may decrease a tumor size by 5 % or more, 10 % or more, 15 % or more, 20 % or more, 25 % or more, 30 % or more, 35 % or more, 45 % or more, 50 % or more, 55 % or more, 65 % or more, 70 % or more, 75 % or more, 80 % or more, 85 % or more, 90 % or more, 100 % or more, about 5 % to about 100 %, about 10 % to about 100 %, about 20 % to about 100 %, about 30 % to about 100 %, about 40 % to about 100 %, about 50 % to about 100 %, about 60 % to about 100 %, about 70 % to about 100 %, about 80 % to about 100 %, or about 90 % to about 100 %, compared to a case in which the microorganisms, or the culture or the extract thereof is not present.

A second aspect provides a pharmaceutical composition for preventing or treating cancer including the microorganisms, or the culture or the extract thereof as an active ingredient.

In the pharmaceutical composition, the cancer may be a solid cancer. The cancer may be a solid cancer existing in a tissue other than a tissue directly contacting the microorganisms when the microorganisms are orally administered. The tissues in direct contact with the microorganisms include the oral cavity, esophagus, stomach, duodenum, small intestine, large intestine, and colon. In addition, tissues other than the tissues in direct contact are breast, lung, head, neck, liver, pancreas, bone, fallopian tube, uterus, vagina, vulva, thyroid, parathyroid, adrenal, soft tissue, urethra, penis, prostate, bladder, kidney, ureter, or central nervous system (CNS). The cancer may be metastatic cancer. The cancer may be, for example, cancers listed in "I. *Enterococcus faecium*", but are not limited thereto.

In the pharmaceutical composition, the composition may be for inhibiting growth of cancer.

In the pharmaceutical composition, the composition may be for co-administering with an immune checkpoint inhibitor. The immune checkpoint inhibitor may be for administering before, concurrently with, or after administration of the microorganisms, or the culture or the extract thereof.

The immune checkpoint inhibitor may be, for example, those listed in "I. *Enterococcus faecium*", but is not limited thereto.

In the pharmaceutical composition, the composition may be for co-administering with a chemotherapeutic agent. The chemotherapeutic agent may be for administering before, concurrently with, or after the administration of the microorganisms, or the culture or the extract thereof.

The chemotherapeutic agent may be, for example, those listed in "I. *Enterococcus faecium*", but is not limited thereto.

In the pharmaceutical composition, the composition may include a pharmaceutically acceptable carrier. The carrier may be a stabilizer, excipient, diluent, or adjuvant. The carrier may be, for example, any and all aqueous and non-aqueous solutions, sterile solutions, solvents, buffers such as a phosphate buffered saline (PBS) solution, water, suspensions, emulsions such as oil/water emulsions, various types of wetting agents, liposomes, dispersion media and coating agents suitable for pharmaceutical administration, particularly suitable for oral administration. The use of such media and agents in pharmaceutical compositions is well known in the art, and compositions including such carriers may be formulated by a well known and common method in the art.

The composition may contain the microorganisms, or the culture or the extract thereof in a "therapeutically effective amount". In the composition, "therapeutically effective amount" means an amount sufficient to exhibit a therapeutic effect when administered to a subject in need of treatment when administered once, or twice or more times. The term "treatment" means treating a cancer disease or medical symptoms of cancer in a mammal, including a human, and includes: alleviating a cancer disease or medical symptoms of cancer; inhibiting a cancer disease or medical symptoms of cancer, that is, slowing or stopping progression of a disease or medical symptoms in a subject; or ameliorating a cancer disease or medical symptoms of cancer in a subject. The "effective amount" may be appropriately selected by a person skilled in the art. The "effective amount" may be about 0.01 wt% to about 50 wt%, or about 0.1 wt% to about 20 wt%, with respect to the weight of the composition. In addition, an amount of the composition administered may be $1 \times 10^6$ CFU/g or more, $1 \times 10^7$ CFU/g or more, $1 \times 10^8$ CFU/g or more, or $1 \times 10^9$ CFU/g or more, with respect to the weight of the composition. For example, the bacterial strain may be administered in an amount of $1 \times 10^{15}$ CFU or less, $1 \times 10^{14}$ CFU or less, $1 \times 10^{13}$ CFU or less, or $1 \times 10^{12}$ CFU or less. For example, the bacterial strain included in the composition may be about $1 \times 10^6$ CFU/g to about $1 \times 10^{15}$ CFU/g, about $1 \times 10^7$ CFU/g to about $1 \times 10^{14}$ CFU/g, about $1 \times 10^8$ CFU/g to about $1 \times 10^{13}$ CFU/g, about $1 \times 10^9$ CFU/g to about $1 \times 10^{12}$ CFU/g, or about $1 \times 10^{10}$ CFU/g to about $1 \times 10^{12}$ CFU/g .

The composition may be administered orally. Accordingly, the composition may be formulated in various forms such as tablets, capsules, liquid formulations such as aqueous solutions, dry syrups or suspensions. For tablets for oral administration, excipients such as lactose and corn starch, and lubricants such as magnesium stearate may be usually added. For capsules for oral administration, lactose and/or dried corn starch may be used as diluents. When oral aqueous suspensions are required, an active ingredient may be combined with emulsifying and/or suspending agents. Also, certain sweetening and/or flavoring agents may be added, when needed. In an embodiment, the composition may be a formulation that allows the microorganisms, or the culture or the extract thereof to be stabilized in an acidic environment such as gastric juice. For example, the composition may be a capsule including the microorganisms, or the culture or the extract thereof therein, or a tablet in which the microorganisms, or the culture or the extract thereof are coated with a film.

A third aspect provides a food composition for preventing or ameliorating cancer including the microorganisms, or the culture or the extract thereof as an active ingredient.

The pharmaceutical composition may include a sitologically acceptable carrier. The carrier may be a stabilizer, excipient, diluent, or adjuvant. The carrier may be, for example, any and all aqueous and non-aqueous solutions, sterile solutions, solvents, buffers such as a phosphate buffered saline (PBS) solution, water, suspensions, emulsions such as oil/water emulsions, various types of wetting agents, liposomes, dispersion media and coating agents suitable for pharmaceutical administration, particularly suitable for oral administration. The use of such media and agents in food compositions is well known in the art, and compositions including such carriers may be formulated by a well-known and common method in the art.

The food may be a dairy product, a soy product, a vegetable and fruit product, or a food additive. The dairy product may be fermented milk, butter, cheese, or powdered milk. The food may be a health functional food. The health functional food may be a health functional food for preventing or improving cancer. The food may be a beverage, confectionery, diet bar, chocolate, pizza, ramen, other noodles, chewing gum, ice cream, and the like.

The food may include ingredients commonly added during food production, for example, proteins, carbohydrates, fats, nutrients, seasonings, and flavors may be included.

The carbohydrates used in food production include monosaccharides such as glucose, fructose, and the like; disaccharides such as maltose, sucrose, oligosaccharides, and the like; and polysaccharides, for example, common sugars such as dextrin, cyclodextrin, and the like, and sugar alcohols such as xylitol, sorbitol, and erythritol. Also, natural flavors and synthetic flavors such as saccharin and aspartame may be used as flavoring agents. The natural flavors may be stevia extracts such as thaumatin, rebaudioside A, and glycyrrhizin. Health functional food refers to a food that brings a specific effect on health when ingested.

In the composition, the microorganisms may be about 0.01 wt% to about 50 wt%, or about 0.1 wt% to about 20 wt%, with respect to the weight of the composition. In addition, an amount of the composition administered may be $1 \times 10^6$ CFU/g or more, $1 \times 10^7$ CFU/g or more, $1 \times 10^8$ CFU/g or more, or $1 \times 10^9$ CFU/g or more, with respect to the weight of the composition. For example, the bacterial strain may be administered in an amount of $1 \times 10^{15}$ CFU or less, $1 \times 10^{14}$ CFU or less, $1 \times 10^{13}$ CFU or less, or $1 \times 10^{12}$ CFU or less. For example, the bacterial strain included in the composition may be about $1 \times 10^6$ CFU/g to about $1 \times 10^{15}$ CFU/g, about $1 \times 10^7$ CFU/g to about $1 \times 10^{14}$ CFU/g, about $1 \times 10^8$ CFU/g to about $1 \times 10^{13}$ CFU/g, about $1 \times 10^9$ CFU/g to about $1 \times 10^{12}$ CFU/g, or about $1 \times 10^{10}$ CFU/g to about $1 \times 10^{12}$ CFU/g.

A fourth aspect provides a method of preventing or treating cancer in a subject including administering the microorganisms, or the culture or the extract thereof in an effective amount for treating cancer.

The subject may be a mammal. The mammal may be a human or a non-human mammal. The administration may be oral administration.

The "effective amount for treating cancer" refers to an amount effective to prevent or treat cancer. The "effective amount for treating cancer" may be about 0.01 mg to about 200 mg of the above-described microorganisms, or the culture or the extract thereof per kg of body weight, or about 0.1 mg to about 400 mg of the microorganisms, or the culture or the extract thereof per kg of body weight. In addition, the "effective amount for treating cancer" administered may be $1 \times 10^6$ CFU or more, $1 \times 10^7$ CFU or more, $1 \times 10^8$ CFU or more, $1 \times 10^9$ CFU per subject. For example, the bacterial strain may be administered in an amount of $1 \times 10^{15}$ CFU or less, $1 \times 10^{14}$ CFU or less, $1 \times 10^{13}$ CFU or less, $1 \times 10^{12}$ CFU or less. For example, the bacterial strain may be administered in an amount of about $1 \times 10^6$ CFU to about $1 \times 10^{15}$ CFU, about $1 \times 10^7$ CFU to about $1 \times 10^{14}$ CFU, about $1 \times 10^8$ CFU to about $1 \times 10^{13}$ CFU, about $1 \times 10^9$ CFU to about $1 \times 10^{12}$ CFU, or about $1 \times 10^{10}$ CFU to about $1 \times 10^{12}$ CFU. For example, a bacterial strain, which is an active ingredient, may be administered twice a day or several times a day in aliquots.

The method may include co-administering with an immune checkpoint inhibitor. The immune checkpoint inhibitor may be for administering before, concurrently with, or after the administration of the microorganisms or the culture or the extract thereof.

The immune checkpoint inhibitor may be, for example, those listed in "I. Enterococcus faecium", but is not limited thereto.

The method may include administering in combination with a chemotherapeutic agent. The chemotherapeutic agent may be for administering before, concurrently with, or after administration of the microorganisms or the culture or the extract thereof.

The chemotherapeutic agent may be, for example, those listed in "I. Enterococcus faecium", but is not limited thereto.

The following represents some preferred embodiments of *Enterococcus faecalis* LMT19-32 microorganisms with anti-tumor activity, but the present disclosure is not limited thereto:

   1. *Enterococcus faecalis* LMT19-32 (accession number KCTC 14306BP) microorganisms having anti-tumor activity.
   2. A pharmaceutical composition for preventing or treating cancer containing the microorganisms, or the culture or the extract thereof of clause 1 as an active ingredient.
   3. The composition according to clause 2, wherein the cancer is a solid cancer.
   4. The composition according to clause 2, which is for co-administering with an immune checkpoint inhibitor.
   5. The composition according to clause 4, wherein the immune checkpoint inhibitor is a CTLA4 inhibitor, PD-1 inhibitor, or PD-L1 inhibitor.
   6. A food composition for preventing or improving cancer containing the microorganisms, or the culture or the extract thereof of clause 1 as an active ingredient.

Hereinafter, the present disclosure will be described in detail by way of examples, but this is only to help understanding of the present disclosure and does not limit the scope of the present disclosure in any way.

## Example

*1. Enterococcus faecium*

**Example 1: Isolation and identification of strains**

1. **Isolation of strains**

For isolation of *Enterococcus faecium* strains, infant feces, adult feces, and food samples were used. In order to isolate *Enterococcus faecium* strains from these samples, the samples were plated on De Man, Rogosa, and Sharpe (MRS) medium (Difco Co., USA) and cultured anaerobically at 30 °C. The samples were taken aseptically, diluted with 180 ml of 0.85 % NaCl solution, and homogenized with a stomacher for 5 minutes. A tube containing 9 ml of 0.85 % NaCl solution was prepared by sterilizing it, and the samples were prepared by diluting in stages in the prepared tube. Thereafter, the samples were spread on an MRS plate medium and cultured at 30 °C for 2 to 3 days, and the colonies that appeared were distinguished by shape and color, and then purified again, and a total of 8 strains were selected. The selected strains were named LMT17-62, LMT17-74, LMT15-24, LMT17-25, LMT15-4, LMT17-43, LMT17-40, and LMT2-17, respectively.

2. **Identification of selected strains**

1) **Analysis of morphological characteristics**

The eight selected strains were cultured in MRS plate medium (Difco, USA), and colony morphology was observed. The colony morphologies of the selected strains are shown in Table 2 below.

[Table 2]

| Morphological characteristics of selected strains | | |
|---|---|---|
| | LMT17-62 | KCTC 13225 |
| Shape | Circular | Circular |
| Size | 0.5 mm | 0.5 mm |
| Color | Cream | Cream |
| Opacity | Opaque | Opaque |
| Elevation | Convex | Convex |
| Surface | Smooth | Smooth |
| Aerobic growth | + | + |
| Anaerobic growth | + | + |

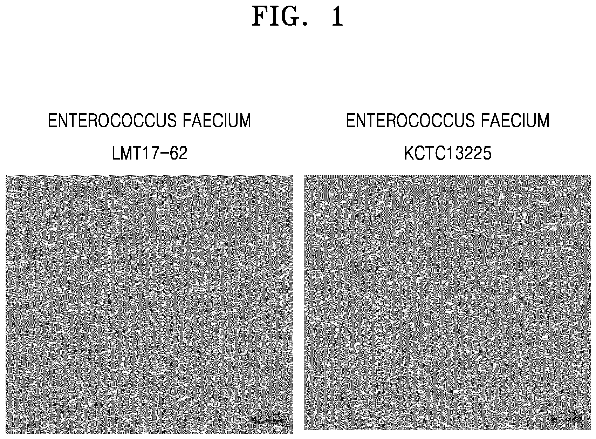

FIG. 1 shows optical microscope images of a strain *Enterococcus faecium* LMT17-62 among the 8 selected strains and a type strain KCTC13225. As shown in FIG. 1, the *Enterococcus faecium* LMT17-62 strain was confirmed to have a spherical shape typical of *Enterococcus* genus.

2) **Analysis of 16S rDNA**

In order to analyze 16S rDNA of the isolated strains, 16S rRNA genes were amplified and sequenced (Macrogen) by using universal bacterial primers (27F (SEQ ID NO: 1) and 1492R (SEQ ID NO: 2). 16S rDNA sequences of the isolated strains are shown in SEQ ID NOS: 3 to 10 (SEQ ID NO: 3: 16S rDNA sequence of LMT17-62; SEQ ID NO: 4: 16S rDNA sequence of LMT17-74; SEQ ID NO: 5: 16S rDNA sequence of LMT15-24; SEQ ID NO: 6: 16S rDNA sequence of LMT17-25; SEQ ID NO: 7: 16S rDNA sequence of LMT15-4; SEQ ID NO: 8: 16S rDNA sequence of LMT17-40; SEQ ID NO: 9: 16S rDNA sequence of LMT2-17; and SEQ ID NO: 10: 16S rDNA sequence of LMT17-43). The results were interpreted by using NCBI blast (http://www.ncbi.nlm.nih.gov/), and the isolated strains were found to belong to a species *Enterococcus faecium.*

The isolated strains were deposited at Korean Collection for Type Cultures (KCTC) at the Korea Research Institute of Bioscience and Biotechnology on August 26, 2020, and finally named *Enterococcus faecium* LMT17-62 (accession number: KCTC 14284BP), *Enterococcus faecium* LMT17-74 (accession number: KCTC 14285BP), *Enterococcus faecium* LMT15-24 (accession number: KCTC 14289BP), *Enterococcus faecium* LMT17-25 (accession number: KCTC 14288BP), *Enterococcus faecium* LMT15-4 (accession number: KCTC 14290BP), *Enterococcus faecium* LMT17-43 (accession number: KCTC 14286BP), *Enterococcus faecium* LMT17-40 (accession number: KCTC 14287BP) and *Enterococcus faecium* LMT2-17 (accession number: KCTC 14291BP), respectively.

**Example 2: Evaluation of anti-tumor efficacy of selected *Enterococcus faecium* strains**

1. **Induction of tumor models in mice and administration of *Enterococcus faecium* strains**

C57BL/6 mice (male, 20 g to 22 g) used as mouse tumor induction models were purchased from Orient Bio Co., Ltd. and were acclimated to the environment for 1 week before the start of the experiment. $2.5 \times 10^5$ MC38 cells derived from colorectal cancer of C57BL/6 mice were injected into a subcutaneous tissue of the mouse back, and after 1 week of tumor injection, groups were separated based on tumor sizes (50 mm³ to 70 mm³). After the group separation, $1.0 \times 10^9$ CFU of *Enterococcus faecium* strain per mouse was orally administered every other day for 2 weeks by using a sonde. For a positive control group, 10 mg of anti-PD1 antibodies (clone number: RMP1-14) per 1 kg of mouse weight was intraperitoneally administered twice a week. For a negative control group, phosphate buffered saline (PBS) was orally administered.

Tumor sizes were measured up to 21 days after the MC38 tumor cell line was injected into the mice, and then the mice were sacrificed by using carbon dioxide, and the tumor and immune organs were extracted to perform an analysis of tumor-infiltrating immune cells.

2. **Evaluation of anti-tumor efficacy according to administration of *Enterococcus faecium* strains**

To investigate tumor suppression efficacy of the *Enterococcus faecium* strains, tumor suppression experiments were conducted on 8 selected microbial strains.

Sizes of the tumor were measured twice a week from day 7 after the injection of the tumor cell line. In order to accurately measure sizes of the tumor, the long axis and the short axis of the tumor were measured by using a Vernier caliper, and the tumor sizes were calculated according to the equation below:

$$\text{Tumor size} = (\text{long axis} \times (\text{short axis})^2)/2.$$

The tumor growth inhibition rates (%) were calculated according to the equation below:

$$\text{Tumor growth inhibition rate (\%)} = (1 - Vt/Vc) \times 100,$$

wherein in the equation, Vc is a mean tumor size in the negative control group when evaluating a tumor size, and

Vt is a mean tumor size in the experimental group when evaluating a tumor size.

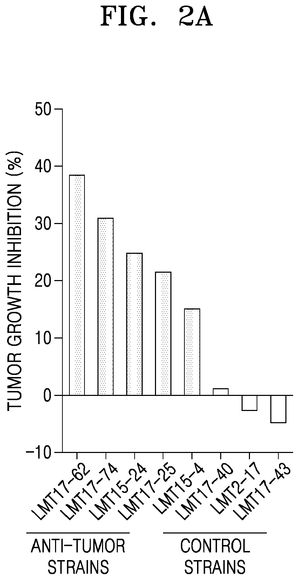

The results are shown in FIG. 2A. As shown in FIG 2A, *Enterococcus faecium* strains LMT17-62, LMT17-74, LMT15-24, LMT17-25, and LMT15-4 showed tumor growth inhibition rates of 39 %, 31 %, 25 %, 22 %, and 15 %, respectively. On the other hand, *Enterococcus faecium* strains LMT17-40, LMT2-17, and LMT17-43 did not show anti-tumor efficacy with tumor growth inhibition rates of 1 %, -3 %, and -5 % or less, respectively. Therefore, it was confirmed that even when strains belong to the same species, there are differences in anti-tumor efficacy depending on the strain.

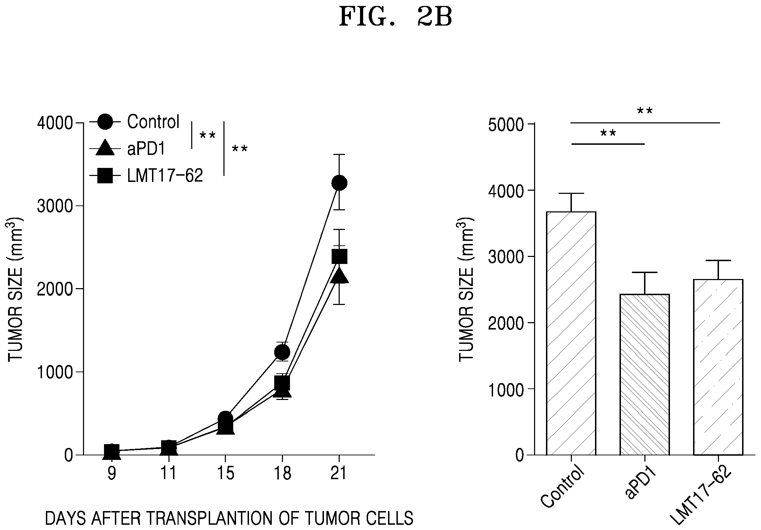

FIG. 2B shows tumor sizes of up to 21 days when the experiment was completed after the injection of the tumor cell line in the group administered with *Enterococcus faecium* LMT17-62 strain. As shown in FIG. 2B, it was confirmed that tumor growth was suppressed statistically significantly in the group administered with *Enterococcus faecium* LMT17-62, and the positive control group administered with anti-PD1 antibodies, compared to the negative control group.

3. **Analysis of tumor-infiltrating immune cells and evaluation of functionality of immune cells**

CD8 T cells, which are tumor-infiltrating immune cells, are important markers of anti-tumor responses, and interferon gamma secreted by CD8 T cells is a functional cytokine that indicates activity of immune cells. In order to confirm whether the anti-tumor efficacy of *Enterococcus faecium* was due to an increase of the above factors, tumors were excised and analyzed after the animal experiments were completed. The excised tumors were separated into single cells by using RPMI1640 medium containing 50 $\mu$g/ml of liberase and 40 $\mu$g/ml of DNase I, and a cell strainer. The isolated cells were stimulated for 4 hours with 50 ng/ml of phorbol 12-myristate 13-acetate (PMA) and 500 ng/ml of ionomycin in RPMI1640 medium containing 10 % fetal bovine serum. After the stimulation, the cells were stained with the antibodies in Table 3 and analyzed by using CANTO II flow cytometry equipment for immune cell analysis and confirmation of interferon gamma production.

[Table 3]

| List of antibodies for flow cytometry | | |
|---|---|---|
| Description | Target | Color |
| Surface marker | CD45 | PE-Cy7 |
| | TCR$\beta$ | APC-Cy7 |
| | CD4 | PerCP-Cy5.5 |
| | CD8 | Pacific blue |
| | PD1 | APC |
| Intracellular staining | IFN-$\gamma$ | FITC |

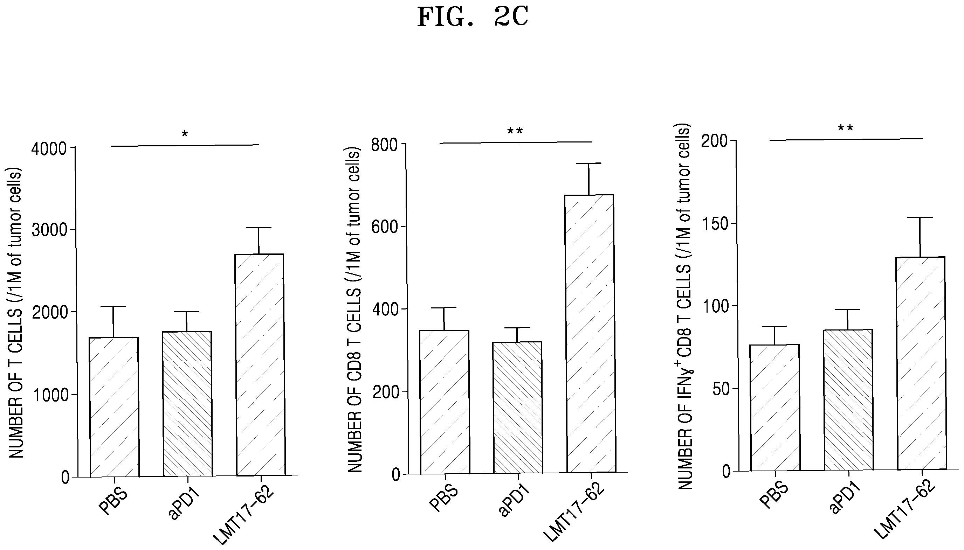

FIG. 2C shows a total number of tumor-infiltrating T cells, CD8 T cells, and interferon gamma-producing CD8 T cells. Tumor-infiltrating T cells and CD8 T cells were significantly increased in a group treated with a LMT17-62 strain compared to a group treated with PBS. In addition, it was confirmed that production of interferon gamma, a CD8 T cell activator, was also significantly increased in the group treated with LMT17-62 compared to the PBS control group.

**Example 3: Analysis of physiological activity characteristics of anti-tumor-positive *Enterococcus faecium* strains**

**1. Evaluation of growth curve and metabolite production ability of anti-tumor-positive *Enterococcus faecium* strains**

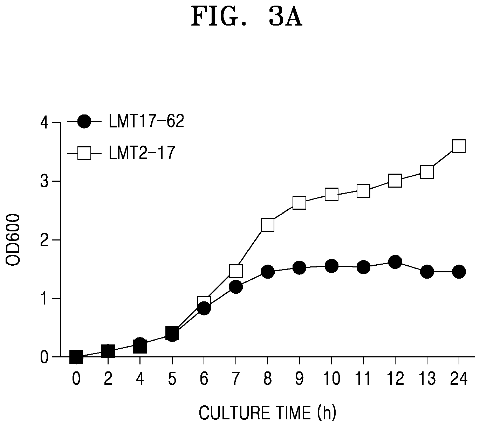

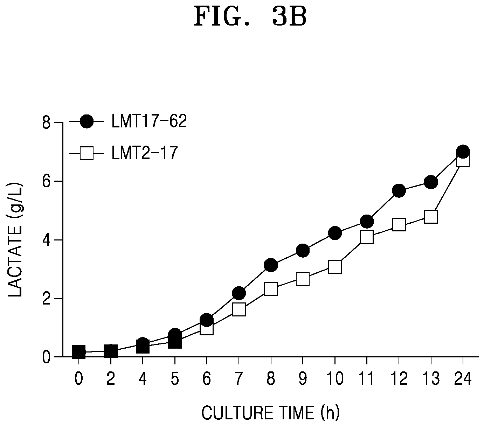

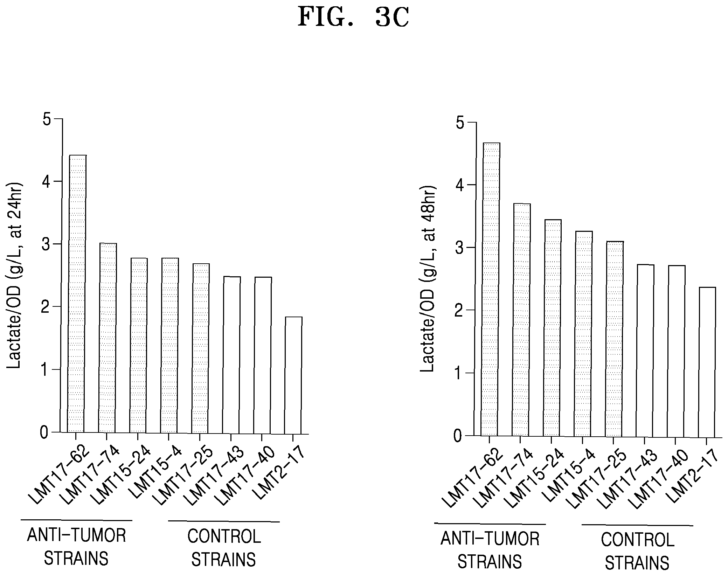

In order to examine growth patterns of the selected *Enterococcus faecium* strains, colonies of anti-tumor-positive strains and anti-tumor-negative strains were cultured in MRS liquid medium for 24 hours, and then the strains were inoculated into 50 ml or 250 ml Erlenmeyer flasks, such that an initial optical density value of each strain at a wavelength of 600 nm ($OD_{600}$) was identical. Then, in order to measure growth curves of the strains, $OD_{600}$ values of the strains were measured every hour by using a spectrophotometer Ultrospec 7000 (biochrom, UK). The results are shown in FIG. 3A. As shown in FIG. 3A, the LMT17-62 strain with anti-tumor efficacy stopped growing after 8 hours of culture, but the anti-tumor negative LMT2-17 strain continued to grow. Amounts of lactate, a metabolite of the strains present in the culture medium, were confirmed by using a biochemical analyzer YSI2900 (Xylem, USA).
The results are shown in FIG. 3B. As shown in FIG. 3B, production of lactate, which is a metabolite, increased as culturing of both the LMT17-62 strain having anti-tumor efficacy and the anti-tumor-negative LMT2-17 strain was continued. FIG. 3C shows abilities to produce lactate, a sugar metabolite, per $OD_{600}$. As shown in FIG 3C, the group of *Enterococcus faecium* strains with anti-tumor efficacy (LMT17-62, LMT17-74, LMT15-24, LMT15-4, and LMT17-25) showed higher capacity to produce lactate per $OD_{600}$ then the group of anti-tumor-negative *Enterococcus faecium* strains (LMT17-43, LMT17-40, and LMT2-17). This was shown more pronounced as the strains were continued to be cultured from 24 hours to 48 hours. That is, when cultured for 48 hours, the anti-tumor-negative strains showed lactate production per $OD_{600}$ of less than 3 g/L, whereas the anti-tumor-positive strains consistently showed lactate production per $OD_{600}$ of 3 g/L or more, and the higher the lactate production ability per $OD_{600}$, the higher the anti-tumor activity.

**2. Evaluation of enzyme activity of anti-tumor-positive *Enterococcus faecium* strains**

In order to investigate biochemical characteristics of the selected *Enterococcus faecium* strains, enzyme activities were evaluated by using a Rapid ID 32A kit (BioMetrieux, France), and the results are shown in Table 4 below. Unlike the group of anti-tumor-negative strains (LMT17-43, LMT17-40, and LMT2-17), the group of *Enterococcus faecium* strains with anti-tumor efficacy (LMT17-62, LMT17-74, LMT15-24, and LMT17-25) had $\beta$-galactosidase enzyme activity.

[Table 4]

| Num ber | Enzyme | Anti-tumor strain | | | | | Control strain | | |
|---|---|---|---|---|---|---|---|---|---|
| | | No.1 | No.2 | No.3 | No.4 | No.5 | No.1 | No.2 | No.3 |
| 1 | Urease | - | - | - | - | - | - | - | - |
| 2 | Arginine Dihydrolase | + | + | + | + | + | + | + | + |
| 3 | $\alpha$-galactosidase | - | - | - | - | - | + | + | + |
| 4 | $\beta$-galactosidase | + | + | + | + | - | - | - | - |
| 5 | $\beta$-galactosidase 6-phosphate | - | - | - | - | - | - | - | - |
| 6 | $\alpha$-glucosidase | - | - | - | - | - | - | - | - |
| 7 | $\beta$-glucosidase | - | - | - | - | - | - | - | - |
| 8 | $\alpha$-arabinosidase | - | - | - | - | - | - | - | - |
| 9 | $\beta$-glucuronidase | - | - | - | - | - | - | - | - |
| 10 | N-acetyl-$\beta$ glucosaminidase | - | - | - | - | - | - | - | - |
| 11 | Mannose fermentation | + | + | + | + | + | + | + | + |
| 12 | Raffinose fermentation | + | - | - | - | - | + | + | + |
| 13 | Glutamic acid decarboxylase | - | - | - | - | - | - | - | - |
| 14 | $\alpha$-fucosidase | - | - | - | - | - | - | - | - |
| 15 | Nitrate reduction | - | - | - | - | - | - | - | - |
| 16 | Indole production | + | + | + | + | + | + | + | + |
| 17 | Alkaline phosphatase | - | - | - | - | - | - | - | - |
| 18 | Arginine arylamidase | + | + | + | + | + | + | + | + |
| 19 | Proline arylamidase | - | - | - | - | - | - | - | - |
| 20 | Leucyl glycine arylamidase | - | - | - | - | - | - | - | - |
| 21 | Phenylalanine arylamidase | + | + | + | + | + | + | + | + |
| 22 | Leucine arylamidase | - | + | + | + | + | + | + | + |
| 23 | Pyroglutamic acid arylamidase | + | + | + | + | + | + | + | + |
| 24 | Tyrosine arylamidase | + | + | + | + | + | + | + | + |
| 25 | Alanine arylamidase | - | - | - | - | - | - | - | - |
| 26 | Glysine arylamidase | - | + | + | + | + | + | + | + |
| 27 | Histidine arylamidase | - | + | + | + | + | + | + | + |
| 28 | Glutamyl glutamic acid arylamidase | - | - | - | - | - | - | - | - |
| 29 | Serine arylamidase | - | + | + | + | + | + | + | + |

In Table 4, the anti-tumor strains No.1, No.2, No.3, No.4, and No.5 are respectively LMT17-62, LMT17-74, LMT15-24, LMT17-25, and LMT15-4, in this order, and control strains No.1, No.2, and No.3 respectively represent LMT17-43, LMT17-40, and LMT2-17, in this order.

### 3. Evaluation of sugar fermentation characteristics of anti-tumor-positive *Enterococcus faecium* strains

In order to analyze sugar metabolism characteristics of the selected *Enterococcus faecium* strains, evaluation was performed by using an API 50 CHL kit (BioMetrieux, France), according to manufacturer's instructions. The results are shown in Table 5. The group of *Enterococcus faecium* strains with anti-tumor efficacy (LMT17-62, LMT17-74, and LMT15-24) had D-sorbitol and D-tagatose sugar fermentation characteristics. In addition, in the case of *Enterococcus faecium* strains (LMT17-62, and LMT17-74) with anti-tumor efficacy, the strains had methyl-αD-mannopyranoside sugar fermentation characteristics.

[Table 5]

| Number | Carbohydrate | Anti-tumor strain | | | | | Control strain | | |
|---|---|---|---|---|---|---|---|---|---|
| | | No.1 | No.2 | No.3 | No.4 | No.5 | No.1 | No.2 | No.3 |
| 1 | Glycerol | - | + | - | - | - | - | - | - |
| 2 | Erythritol | - | - | - | - | - | - | - | - |
| 3 | D-arabinose | - | - | - | - | - | - | - | - |
| 4 | L-arabinose | + | + | + | + | + | + | + | + |
| 5 | D-ribose | + | + | + | + | + | + | + | + |
| 6 | D-xylose | - | - | - | - | - | - | - | - |
| 7 | L-xylose | - | - | - | - | - | - | - | - |
| 8 | D-adonitol | - | - | - | - | - | - | - | - |
| 9 | Methyl-βD-xylopyranoside | - | - | - | - | - | - | - | - |
| 10 | D-galactose | + | + | + | + | + | + | + | + |
| 11 | D-glucose | + | + | + | + | + | + | + | + |
| 12 | D-fructose | + | + | + | + | + | + | + | + |
| 13 | D-mannose | + | + | + | + | + | + | + | + |
| 14 | L-sorbose | - | - | - | - | - | - | - | - |
| 15 | L-rhamnose | - | - | - | + | - | - | + | - |
| 16 | Dulcitol | + | - | - | - | - | - | - | - |
| 17 | Inositol | - | - | - | - | - | - | - | - |
| 18 | D-mannitol | + | + | + | + | + | + | + | + |
| 19 | D-sorbitol | + | + | + | - | - | - | - | - |
| 20 | Methyl-αD-mannopyranoside | + | + | - | - | - | - | - | - |
| 21 | Methyl-αD-glucopyranoside | - | - | - | - | - | - | - | - |
| 22 | N-acetylglucosamine | + | + | + | + | + | + | + | + |
| 23 | Amygdalin | + | + | + | + | - | + | + | + |
| 24 | Arbutin | + | + | + | + | + | + | + | + |
| 25 | Aesculin | + | + | + | + | + | + | + | + |
| 26 | Salicin | + | + | + | + | + | + | + | + |
| 27 | D-cellobiose | + | + | + | + | + | + | + | + |
| 28 | D-maltose | + | + | + | + | + | + | + | + |
| 29 | D-lactose | + | + | + | + | + | + | + | + |
| 30 | D-melibiose | + | + | + | - | + | + | + | + |
| 31 | D-saccharose | + | + | + | + | + | + | + | + |
| 32 | D-trehalose | + | + | + | + | + | + | + | + |
| 33 | Inulin | - | - | - | - | - | - | - | - |
| 34 | D-melezitose | - | + | - | - | - | - | - | - |

(continued)

| Number | Carbohydrate | Anti-tumor strain | | | | | Control strain | | |
|---|---|---|---|---|---|---|---|---|---|
| | | No.1 | No.2 | No.3 | No.4 | No.5 | No.1 | No.2 | No.3 |
| 35 | D-raffinose | + | - | + | - | - | + | + | + |
| 36 | Amidone | - | - | - | - | - | - | - | - |
| 37 | Glycogen | - | - | - | - | - | - | - | - |
| 38 | Xylitol | - | - | - | - | - | - | - | - |
| 39 | Gentiobios | + | + | + | + | + | + | + | + |
| 40 | D-turanose | - | - | - | - | - | - | - | - |
| 41 | D-lyxose | - | - | - | - | - | - | - | - |
| 42 | D-tagatose | + | + | + | - | - | - | - | - |
| 43 | D-fucose | - | - | - | - | - | - | - | - |
| 44 | L-fucose | - | - | - | - | - | - | - | - |
| 45 | D-arabitol | - | - | - | - | - | - | - | - |
| 46 | L-arabitol | - | - | - | - | - | - | - | - |
| 47 | Potassium gluconate | + | + | + | + | - | - | + | - |
| 48 | Potassium 2-ketogluconate | - | - | - | - | - | - | - | - |
| 49 | Potassium 5-ketogluconate | - | - | - | - | - | - | - | - |

In Table 5, the anti-tumor strains No.1, No.2, No.3, No.4, and No.5 are respectively LMT17-62, LMT17-74, LMT15-24, LMT17-25, and LMT15-4, in this order, and control strains No.1, No.2, and No.3 respectively represent LMT17-43, LMT17-40, and LMT2-17, in this order.

## 4. Evaluation of stability of anti-tumor-positive *Enterococcus faecium* strains

### (1) Investigation of acid resistance

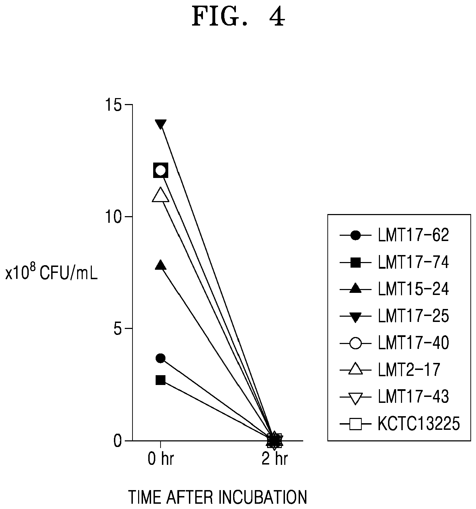

In order to evaluate acid resistance of *Enterococcus faecium* strains, an experiment was conducted in the following manner. The selected strains were inoculated into sterilized MRS liquid medium and then cultured at 37 °C for 16 hours. Then, after inoculating the strains into sterilized MRS liquid medium, in which pH was adjusted to 2.5 by using HCl, the cells were incubated at 37 °C for 2 hours, and numbers of live bacteria were confirmed. Live bacteria before inoculation and live bacteria at 2 hours after the inoculation were spread on MRS plate medium and then cultured, and numbers of colonies thereof were counted and compared, and the results are shown in FIG. 4.

As shown in FIG. 4, since the anti-tumor-positive *Enterococcus faecium* strain was shown to have somewhat low acid resistance, it may be preferable to use a capsule or a coating agent during formulation.

### (2) Examination of bile resistance

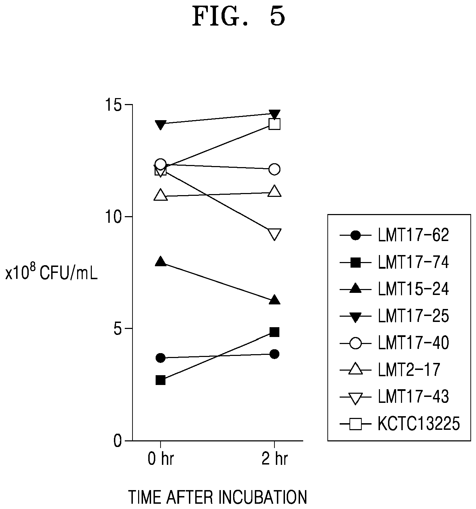

In order to examine bile resistance of *Enterococcus faecium* strains, an experiment was conducted in the following manner. The selected strains were inoculated into sterilized MRS liquid medium and then cultured at 37 °C for 16 hours. Considering that a concentration of bile salts in the intestinal tract is around 0.1 %, only the bacterial cells were inoculated into MRS liquid medium containing 0.3 % of bile salts (Sigma, USA) to $10^8$ CFU/ml to $10^9$ CFU/ml, and cultured at 37 °C for 2 hours, then numbers of live bacteria were identified. Live bacteria before inoculation and live bacteria at 2 hours after the inoculation were spread on MRS plate medium and then cultured to count numbers of colonies thereof. The results are shown in FIG. 5.

As shown in FIG. 5, since the anti-tumor-positive *Enterococcus faecium* strains maintained an appropriate number of bacteria even at 0.3 %, which is higher than 0.1 %, which is similar to the actual concentration in the intestine, it may be determined that the selected anti-tumor-positive *Enterococcus faecium* strains may survive sufficiently in the intestines of humans or animals.

**(3) Investigation of intestinal anchorage**

In order to evaluate intestinal cell anchorage of the anti-tumor-positive *Enterococcus faecium* strains, a Caco-2 cell line (KCLB 30037.1), which is human epithelial colorectal adenocarcinoma cells purchased from the Korea Cell Line Bank, was used. Caco-2 cells were divided in Dulbecco's modified Eagle's medium (DMEM; Gibco, USA) containing 10 % fetal bovine serum (FBS) (Gibco, USA) under conditions of 5 % COz and 37 °C, to be $7 \times 10^4$ cells/100 μl, and cultured to form a monolayer of cells in a 96-well plate (Corning, USA).

On the other hand, the selected anti-tumor-positive *Enterococcus faecium* strains cultured in MRS liquid medium were washed with phosphate buffered saline (PBS), suspended in antibiotic-free DMEM medium, and added to Caco-2 cells that formed a monolayer of cells, so that amounts of the *Enterococcus faecium* strains would be $1 \times 10^7$ CFU, and the selected anti-tumor-positive *Enterococcus faecium* strains were cultured for 2 hours under conditions of 5 % $CO_2$ and 37 °C. In order to remove cells that failed to adhere to the Caco-2 cells, the cells were washed 5 times with PBS, and the adhered cells were detached with 100 μl of 0.1 % Triton x-100 and then spread on MRS solid medium. After incubation at 37 °C for 24 hours, numbers of colonies on the plate medium were counted to examine intestinal anchorage of the *Enterococcus faecium* strains.

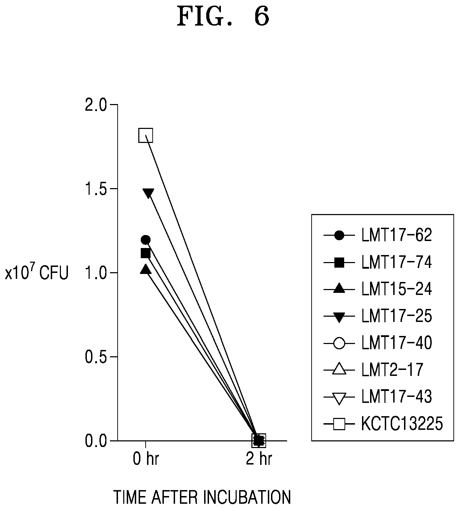

FIG. 6 shows numbers of the selected anti-tumor-positive *Enterococcus faecium* strains attached to intestinal epithelial cells. As shown in FIG. 6, it was confirmed that LMT17-62, LMT17-74, LMT15-24, and LMT17-25 had intestinal anchorage on intestinal epithelial cells (Caco-2) of 0.31 %, 1.55 %, 0.46 %, and 0.48 %, respectively.

**II. *Enterococcus faecalis* LMT19-32**

**Example 1: Isolation and identification of *Enterococcus faecalis* LMT19-32 strain**

**1. Isolation of *Enterococcus faecalis* strains**

*Enterococcus faecalis* strains were isolated from adult fecal samples. First, the samples were spread on MRS medium (Difco Co., USA) and cultured anaerobically at 30 °C. As a pretreatment method of the sample, the sample was taken aseptically, diluted with 180 ml of 0.85 % NaCl solution, and the fecal solution was homogenized by using a stomacher for 5 minutes. A fecal sample was prepared by diluting the homogenized sample in stages in a tube containing 9 ml of a sterile 0.85 % NaCl solution. The sample was spread on MRS plate medium (Difco, USA) and cultured at 37 °C for 2 days to 3 days, and the colonies that appeared were distinguished according to shape and color, and then purified and separated again.

**2. Identification of *Enterococcus faecalis* strains**

**(1) Analysis of morphological characteristics**

A selected strain was cultured in MRS plate medium (Difco, USA), and colony morphology was observed. Colony morphology of the selected *Enterococcus faecalis* strain and the *Enterococcus faecalis* type strain KCTC3206, on the MRS plate medium is shown in Table 6 below.

[Table 6]

|  | LMT19-32 | KCTC3206 |
|---|---|---|
| Shape | Circular | Circular |
| Size | 0.8mm | 0.8mm |
| Color | Cream | Cream |
| Transparency | Opaque | Opaque |
| Elevation | Convex | Convex |
| Surface | Smooth | Smooth |
| Aerobic growth | + | + |
| Anaerobic growth | + | + |

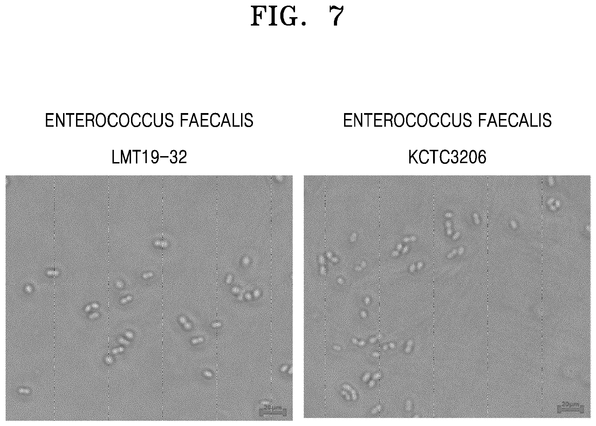

FIG. 7 shows representative optical microscope images of the selected strain *Enterococcus faecalis* LMT19-32 and the type strain KCTC3206. As shown in FIG. 7, the LMT19-32 strain was coccus and was similar in morphology to a typical *Enterococcus* genus.

**(2) Analysis of 16S rDNA**

16S rRNA genes of the isolated LMT19-32 strain were amplified, and a nucleotide sequence of the amplified 16S rRNA genes was analyzed. The amplification was performed by PCR by using genomic DNA of the LMT19-32 strain as a template and an oligonucleotide of SEQ ID NO: 11 and an oligonucleotide of SEQ ID NO: 12 (Macrogen) as a primer set. The nucleotide sequence of 16S rDNA of the isolated LMT19-32 strain is shown in SEQ ID NO: 13. The nucleotide sequence of the identified 16S rDNA was compared with the nucleotide sequence of the known 16S rDNA by using NCBI blast (http://www.ncbi.nlm.nih.gov/). As a result, the 16S rDNA of LMT19-32 had 100 % sequence identity with that of the *Enterococcus faecalis* species. In addition, as a result of phylogenetic tree analysis, LMT19-32 was the same as the *Enterococcus faecalis* species. As a result, LMT19-32 strain was identified as a new strain belonging to a *Enterococcus faecalis* species.

The present inventors named LMT19-32 lactic acid bacteria as *"Enterococcus faecalis* LMT19-32" (accession number: KCTC14306BP) and deposited the same to the Korean Collection for Type Cultures (KCTC) at the Korea Research Institute of Bioscience and Biotechnology, on September 9, 2020.

**Example 2: Analysis of physiological activity characteristics of *Enterococcus faecalis* LMT19-32 strain**

**1. Sugar fermentation characteristics of strain *Enterococcus faecalis* LMT19-32**

Sugar metabolism characteristics of the selected LMT19-32 strain were confirmed by using an API 50 CHL kit (BioMetrieux, France) according to the manufacturer's experimental method. Table 7 shows sugar fermentation characteristics of the identified LMT19-32 strain.

[Table 7]

| Number | Carbohydrate | LMT19-32 | |
|---|---|---|---|
| | | 24 hours | 48 hours |
| 1 | Glycerol | + | + |
| 2 | Erythritol | - | - |
| 3 | D-arabinose | - | - |
| 4 | L-arabinose | - | - |
| 5 | D-ribose | + | + |
| 6 | D-xylose | - | - |
| 7 | L-xylose | - | - |
| 8 | D-adonitol | - | - |
| 9 | Methyl-bD-xylopyranoside | - | - |
| 10 | D-galactose | + | + |
| 11 | D-glucose | + | + |
| 12 | D-fructose | + | + |
| 13 | D-mannose | + | + |
| 14 | L-sorbose | - | - |
| 15 | L-rhamnose | - | - |
| 16 | Dulcitol | - | - |
| 17 | Inositol | - | + |
| 18 | Mannitol | + | + |
| 19 | D-sorbitol | + | + |

(continued)

| Number | Carbohydrate | LMT19-32 | |
|---|---|---|---|
| | | 24 hours | 48 hours |
| 20 | Methyl-αD-mannopyranoside | - | - |
| 21 | Methyl-αD-glucopyranoside | - | - |
| 22 | N-acetylglucosamine | + | + |
| 23 | Amygdalin | + | + |
| 24 | Arbutin | + | + |
| 25 | Aesculin | + | + |
| 26 | Salicin | + | + |
| 27 | D-cellobiose | + | + |
| 28 | D-maltose | + | + |
| 29 | D-lactose | + | + |
| 30 | D-melibiose | - | - |
| 31 | D-saccharose | + | + |
| 32 | D-trehalose | + | + |
| 33 | Inulin | - | - |
| 34 | D-melezitose | + | + |
| 35 | D-raffinose | - | - |
| 36 | Amidone | - | - |
| 37 | Glycogen | - | - |
| 38 | Xylitol | - | - |
| 39 | Gentiobios | + | + |
| 40 | D-turanose | - | - |
| 41 | D-lyxose | - | - |
| 42 | D-tagatose | + | + |
| 43 | D-fucose | - | - |
| 44 | L-fucose | - | - |
| 45 | D-arabitol | - | - |
| 46 | L-arabitol | - | - |
| 47 | Potassium gluconate | + | + |
| 48 | Potassium 2-ketogluconate | - | - |
| 49 | Potassium 5-ketogluconate | - | - |

2. **Evaluation of stability of *Enterococcus faecalis* LMT19-32 strain**

In order to evaluate acid resistance of *Enterococcus faecalis* LMT19-32 strain, an experiment was conducted in the following manner. The LMT19-32 strain was inoculated into sterilized MRS liquid medium and then cultured at 37 °C for 16 hours. Then, 1 % of the strain was inoculated into a sterilized MRS liquid medium adjusted to pH 2.5 with HCl, and cultured at 37 °C for 2 hours. Samples immediately after the strain inoculation and after 2 hours of incubation were collected, diluted in MRS liquid medium, spread on MRS plate medium, incubated at 37 °C for 24 hours, and then the number of colonies on the plate medium was counted to measure the number of bacteria.

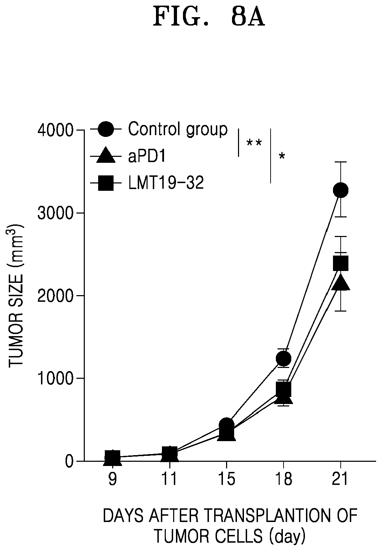

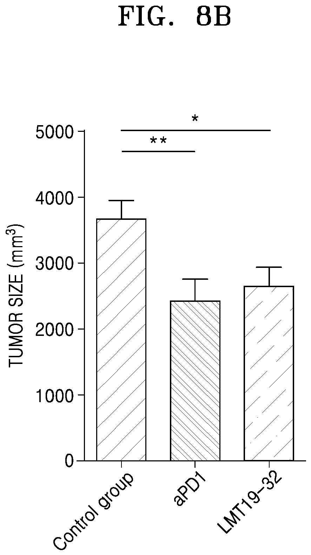

As shown in FIG. 8, since the *Enterococcus faecalis* LMT19-32 strain was shown to have somewhat low acid resistance, it may be preferable to use a capsule or a coating agent during formulation.

[Table 8]

|  | LMT19-32 | KCTC3206 |
|---|---|---|
| MRS (pH 6.8) (cell number/plate) | $3.1 \times 10^9$ | $1.8 \times 10^9$ |
| MRS (pH 2.5) (cell number/plate) | $3.0 \times 10^1$ | $8.5 \times 10^4$ |
| Survival rate (%) | 0.000001 | 0.004670 |

(2) Examination of bile resistance

In order to confirm effects of bile acid on the *Enterococcus faecalis* LMT19-32 strain, an experiment was conducted in the following manner. The selected strain were inoculated into sterilized MRS liquid medium and then cultured at 37 °C for 24 hours. Considering that a concentration of bile salts in the intestine is around 0.1 %, 1 % of the strain was inoculated into MRS liquid medium containing 0.3 % of bile salts (Sigma, USA) and cultured at 37 °C for 2 hours. Samples immediately after the strain inoculation and after 2 hours of incubation were collected, diluted in MRS liquid medium, spread on MRS plate medium, incubated at 37 °C for 24 hours, and then the number of colonies on the plate medium was counted to measure the number of viable cells of the strain. As a control group, culturing was performed in the same manner in MRS liquid medium without 0.3 % bile salts, and the number of viable cells of the strain was counted. Table 9 shows results of measuring bile salt resistance. As shown in Table 9, the LMT19-32 strain maintained a survival rate of 93 % at 0.3 %, which is higher than 0.1 %, which is similar to the actual concentration in the intestine, and therefore, LMT19-32 strain may be determined to survive in the intestine of humans or animals.

[Table 9]

|  | LMT19-32 | KCTC3206 |
|---|---|---|
| Control group (cell number/plate) | $3.1 \times 10^9$ | $1.8 \times 10^9$ |
| 0.3 % bile salt (cell number/plate) | $2.9 \times 10^9$ | $1.4 \times 10^9$ |
| Survival rate (%) | 93.0 | 80.2 |

**(3) Investigation of intestinal anchorage**

In order to evaluate intestinal cell anchorage of the *Enterococcus faecalis* LMT19-32 strain, a Caco-2 cell line (KCLB 30037.1), which is human epithelial colorectal adenocarcinoma cells purchased from the Korea Cell Line Bank, was used. Caco-2 cells were divided in Dulbecco's modified Eagle's medium (DMEM; Gibco, USA) containing 10 % fetal bovine serum (FBS) (Gibco, USA) under conditions of 5 % $CO_2$ and 37 °C, to be $7 \times 10^4$ cells/100 μl, and cultured to form a monolayer of cells in a 96-well plate (Corning, USA).

On the other hand, the LMT19-32 strain cultured in MRS liquid medium was washed with PBS, then suspended in DMEM medium to which antibiotics are not added, and the LMT19-32 strain was added to Caco-2 cells forming a monolayer of cells to $1 \times 10^7$ CFU, and was incubated for 2 hours under conditions of 5 % $CO_2$ and 37 °C. In order to remove cells that failed to adhere to the Caco-2 cells, the cells were washed 5 times with PBS, and the adhered cells were detached with 100 μl of 0.1 % Triton x-100 and then spread on MRS solid medium. After incubation at 37 °C for 24 hours, numbers of colonies on the plate medium were counted to examine intestinal anchorage of the LMT19-32 strain.

Table 10 is a diagram showing a number of colonies attached to intestinal epithelial cells of *Enterococcus faecalis* LMT19-32 strain. As shown in Table 10, it was confirmed that both the novel *Enterococcus faecalis* LMT19-32 strain of the present disclosure and the comparative strain *Enterococcus faecalis* KCTC3206 had the ability to anchor to Caco-2, which is intestinal epithelial cells of about 1 %.

[Table 10]

|  | LMT19-32 | KCTC3206 |
|---|---|---|
| Number of treated strains ($10^7$ CFU) | 1.4 | 1.5 |
| Number of anchored strains ($10^4$ CFU) | 10.4 | 19.4 |
| Anchorage rate (%) | 0.75 | 1.26 |

**Example 3: Analysis of anti-tumor efficacy of *Enterococcus faecalis LMT19*-32 strain 1. Induction of tumor models in mice and administration of *Enterococcus faecalis* LMT19-32 strain**

C57BL/6 mice (male, 20 g to 22 g) used as mouse tumor induction models were purchased from Orient Bio Co., Ltd. and were acclimated to the environment for 1 week before the experiment began. $2.5 \times 10^5$ MC38 cells derived from colorectal cancer of C57BL/6 mice were injected into a subcutaneous tissue of the mouse back, and after 1 week of tumor injection, groups were separated based on tumor sizes (50 mm³ to 70 mm³). After the group separation, PBS containing $1.0 \times 10^9$ CFU of the *Enterococcus faecalis LMT19-32* strain per mouse was orally administered every other day for 2 weeks by using a sonde. For a positive control group, 10 mg of anti-PD1 antibodies (clone number: RMP1-14, manufacturer: Bioxcell, product name: InvivoMAb anti-mouse PD-1) per 1 kg of mouse weight was intraperitoneally administered twice a week. As a negative control group, PBS was orally administered. The experimental groups were configured as shown in Table 11, with a total of 3 groups of 10 animals in each group.

[Table 11]

| Group 1 | PBS control group |
|---------|-------------------|
| Group 2 | Anti-PD1 antibodies-treated group |
| Group 3 | *Enterococcus faecalis* LMT19-32-treated group |

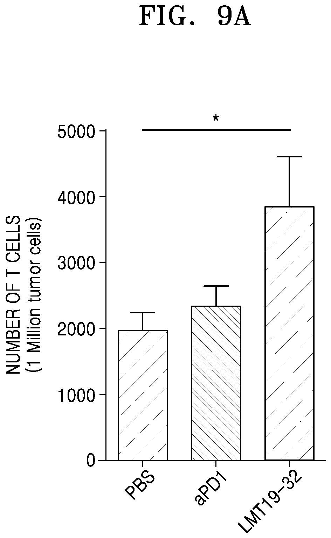

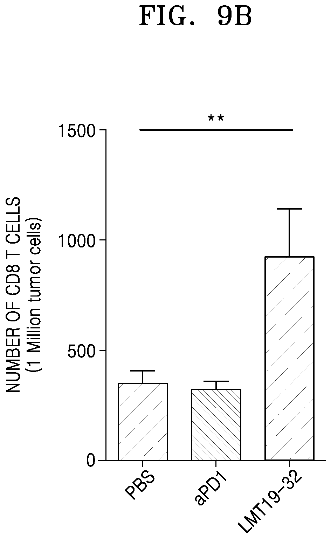

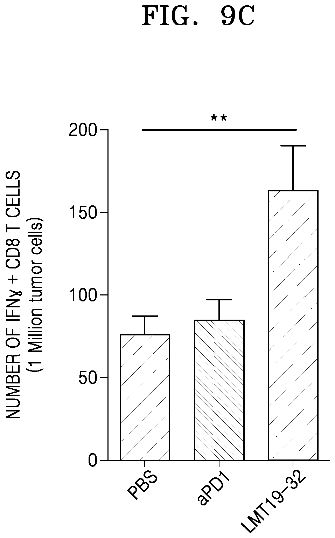

Tumor sizes were measured up to 21 days after the MC38 tumor cell line was injected into the mice, and then the mice were sacrificed by using carbon dioxide, and the tumor was extracted to perform an analysis of tumor-infiltrating immune cells. The analysis results are shown in FIG. 9A, 9B and 9C. FIGS. 9A, 9B, and 9C show results of analyzing tumor-infiltrating T cells, CD8 T cells, and IFNγ⁺ CD8 T cells, which express IFNγ, after the administration of the *Enterococcus faecalis LMT19-32* strain to mice with tumor. The MC38 tumor cell line is mouse colon adenocarcinoma cells induced by subcutaneous injection of dimethylhydrazine into C57BL/6 mice. Since the mouse tumor-induced models used in this example had MC38 tumor cells transplanted into the subcutaneous tissue, it is indicated that an effect of the orally administered strain inhibits cancer growth in tissues other than tissues directly contacted by the strain, such as subcutaneous tissue, and that the effect of the bacteria may not only act on cancers associated with the gastrointestinal tract or intestine, which is a tissue in direct contact with the strain, but also on various solid carcinomas.

**2. Evaluation of anti-tumor efficacy according to administration of *Enterococcus faecalis* LMT19-32 strain**

FIGS. 8A and 8B are diagrams showing results of measuring tumor sizes after administering the *Enterococcus faecalis* LMT19-32 strain to mice with tumor. The administration was performed according to Section 1. Specifically, sizes of the tumor were measured twice a week from day 7 after the injection of the tumor cell line to day 21. In order to accurately measure the sizes of the tumor, the long axis and the short axis of the tumor were measured by using a Vernier caliper, and the tumor sizes were calculated according to an equation of 'Tumor size = (long axis × (short axis)²)/2'. In FIGS. 8A and 8B, the negative control group administered PBS, the aPD1 group administered anti-PD1 antibodies as a positive control group, and the LMT19-32 group administered LMT19-32 as an experimental group. The graph of FIG. 8A shows tumor sizes according to a number of days after the cell injection, and FIG. 8B shows tumor sizes on day 21 after the cell injection. In FIG. 8A, the horizontal axis represents days after the tumor cell administration on which tumor sizes were measured. In FIG. 8B, the bars indicate all tumor sizes of each group on day 21 after the cell injection. As shown in FIGS. 8A and 8B, statistically significant tumor growth inhibition was observed in the group administered with LMT19-32 strain alone.

**3. Analysis of tumor-infiltrating immune cells and evaluation of functionality of immune cells**

CD8 T cells, which are tumor-infiltrating immune cells, are important markers of anti-cancer responses, and interferon gamma and granzyme B secreted by CD8 T cells are functional cytokines that indicate activity of immune cells. FIGS. 9A, 9B, and 9C show results of analyzing tumor-infiltrating T cells, and cytokine secretion thereby, after administration of the *Enterococcus faecalis* LMT19-32 strain to mice with tumor. The administration was performed according to Section 1. Tumors were excisedd 21 days after the administration. The excised tumors were separated into single cells by using RPMI1640 medium containing 50 μg/ml of liberase and 40 μg/ml of DNase I, and a cell strainer. In order to observe interferon gamma production pattern of T cells, the isolated cells were stimulated for 4 hours with 50 ng/ml of phorbol 12-myristate 13-acetate (PMA) and 500 ng/ml of ionomycin in RPMI1640 medium containing 10 % FBS. PMA and ionomycin are substances that give signal stimulations for activating T cells, and bring the same results as an activation mechanism of T cells by actual antigens, creating an environment in which an immune response occurs.

Ionomycin increases protein kinase C (PKC) as a $Ca^{2+}$ ionophore, and PMA plays a synergizing role by phosphorylating PKC to target CD4 T cells and CD8 T cells. After the stimulation, the cells were stained with the antibodies in Table 12 and analyzed by using CANTO II flow cytometry equipment for immune cell analysis and confirmation of interferon gamma production.

[Table 12]

| Description | Target | Color |
|---|---|---|
| Surface marker | CD45 | PE-Cy7 |
| | TCRβ | APC-Cy7 |
| | CD4 | PerCP-Cy5.5 |
| | CD8 | Pacific blue |
| Intracellular staining | IFNγ | FITC |
| | Granzyme B | APC |

FIGS. 9A, 9B, and 9C show total numbers of tumor-infiltrating T cells, numbers of CD8 T cells among tumor-infiltrating T cells, and numbers of IFNγ-expressing cells among CD8 T cells, respectively. As shown in FIG. 9, a number of tumor-infiltrating T cells and a number of CD8 T cells increased significantly in the group administered with LMT19-32 compared to the PBS-treated group. In addition, a number of CD8 T cells producing interferon gamma increased significantly in the LMT19-32-treated group compared to the PBS control group.

# EP 4 206 317 A1

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

## RECEIPT IN THE CASE OF AN ORIGINAL DEPOSIT

issued pursuant to Rule 7.1

TO: Medytox Inc.

  Medytox Inc.

  114, Central town-ro, Yeongtong-gu, Suwon-si, Gyeonggi-do

  Republic of Korea

| I. IDENTIFICATION OF THE MICROORGANISM | |
|---|---|
| Identification reference given by the DEPOSITOR: <br><br> *Enterococcus faecium* **LMT17-62** | Accession number given by the INTERNATIONAL DEPOSITARY AUTHORITY: <br><br> **KCTC 14284BP** |

| II. SCIENTIFIC DESCRIPTION AND/OR PROPOSED TAXONOMIC DESIGNATION |
|---|
| The microorganism identified under I above was accompanied by: <br><br> [ ] a scientific description <br><br> [ ] a proposed taxonomic designation <br><br> (Mark with a cross where applicable) |

| III. RECEIPT AND ACCEPTANCE |
|---|
| This International Depositary Authority accepts the microorganism identified under I above, which was received by it on **August 26, 2020.** |

| IV. RECEIPT OF REQUEST FOR CONVERSION |
|---|
| The microorganism identified under I above was received by this International Depositary Authority on and a request to convert the original deposit to a deposit under the Budapest Treaty was received by it on |

| V. INTERNATIONAL DEPOSITARY AUTHORITY | |
|---|---|
| Name: **Korean Collection for Type Cultures** <br><br> Address: Korea Research Institute of <br> Bioscience and Biotechnology (KRIBB) <br> 181, Ipsin-gil, Jeongeup-si, Jeollabuk-do 56212 <br> Republic of Korea | Signature(s) of person(s) having the power to represent the International Depositary Authority or of authorized official(s): <br><br> *Song-Gun Kim* <br><br> KIM, Song-Gun, Director <br> Date: **September 9, 2020** |

Form BP/4 (KCTC Form 17)                     sole page
                                         (Reissue)

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

## RECEIPT IN THE CASE OF AN ORIGINAL DEPOSIT

issued pursuant to Rule 7.1

TO: Medytox Inc.

Medytox Inc.

114, Central town-ro, Yeongtong-gu, Suwon-si, Gyeonggi-do

Republic of Korea

| I. IDENTIFICATION OF THE MICROORGANISM | |
|---|---|
| Identification reference given by the DEPOSITOR:<br><br>*Enterococcus faecium* **LMT17-74** | Accession number given by the INTERNATIONAL DEPOSITARY AUTHORITY:<br><br>**KCTC 14285BP** |

| II. SCIENTIFIC DESCRIPTION AND/OR PROPOSED TAXONOMIC DESIGNATION |
|---|
| The microorganism identified under I above was accompanied by:<br><br>[ ] a scientific description<br><br>[ ] a proposed taxonomic designation<br><br>(Mark with a cross where applicable) |

| III. RECEIPT AND ACCEPTANCE |
|---|
| This International Depositary Authority accepts the microorganism identified under I above, which was received by it on **August 26, 2020.** |

| IV. RECEIPT OF REQUEST FOR CONVERSION |
|---|
| The microorganism identified under I above was received by this International Depositary Authority on and a request to convert the original deposit to a deposit under the Budapest Treaty was received by it on |

| V. INTERNATIONAL DEPOSITARY AUTHORITY | |
|---|---|
| Name: **Korean Collection for Type Cultures**<br><br>Address: Korea Research Institute of<br>Bioscience and Biotechnology (KRIBB)<br>181, Ipsin-gil, Jeongeup-si, Jeollabuk-do 56212<br>Republic of Korea | Signature(s) of person(s) having the power to represent the International Depositary Authority or of authorized official(s):<br><br>*Song-Gun Kim*<br><br>KIM, Song-Gun, Director<br>Date: **September 9, 2020** |

Form BP/4 (KCTC Form 17)                    sole page
(Reissue)

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

## RECEIPT IN THE CASE OF AN ORIGINAL DEPOSIT

issued pursuant to Rule 7.1

TO: Medytox Inc.

Medytox Inc.

114, Central town-ro, Yeongtong-gu, Suwon-si, Gyeonggi-do

Republic of Korea

---

**I. IDENTIFICATION OF THE MICROORGANISM**

| Identification reference given by the DEPOSITOR: | Accession number given by the INTERNATIONAL DEPOSITARY AUTHORITY: |
|---|---|
| ***Enterococcus faecium* LMT17-43** | **KCTC 14286BP** |

**II. SCIENTIFIC DESCRIPTION AND/OR PROPOSED TAXONOMIC DESIGNATION**

The microorganism identified under I above was accompanied by:

[ ] a scientific description

[ ] a proposed taxonomic designation

(Mark with a cross where applicable)

**III. RECEIPT AND ACCEPTANCE**

This International Depositary Authority accepts the microorganism identified under I above, which was received by it on **August 26, 2020.**

**IV. RECEIPT OF REQUEST FOR CONVERSION**

The microorganism identified under I above was received by this International Depositary Authority on and a request to convert the original deposit to a deposit under the Budapest Treaty was received by it on

**V. INTERNATIONAL DEPOSITARY AUTHORITY**

| Name: **Korean Collection for Type Cultures** | Signature(s) of person(s) having the power to represent the International Depositary Authority or of authorized official(s): |
|---|---|
| Address: Korea Research Institute of Bioscience and Biotechnology (KRIBB) 181, Ipsin-gil, Jeongeup-si, Jeollabuk-do 56212 Republic of Korea | *Song-Gun Kim* KIM, Song-Gun, Director Date: **September 9, 2020** |

Form BP/4 (KCTC Form 17)

sole page

(Reissue)

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

## RECEIPT IN THE CASE OF AN ORIGINAL DEPOSIT

issued pursuant to Rule 7.1

TO: Medytox Inc.

Medytox Inc.

114, Central town-ro, Yeongtong-gu, Suwon-si, Gyeonggi-do

Republic of Korea

| I. IDENTIFICATION OF THE MICROORGANISM | |
|---|---|
| Identification reference given by the DEPOSITOR:<br><br>***Enterococcus faecium* LMT17-40** | Accession number given by the INTERNATIONAL DEPOSITARY AUTHORITY:<br><br>**KCTC 14287BP** |

| II. SCIENTIFIC DESCRIPTION AND/OR PROPOSED TAXONOMIC DESIGNATION |
|---|
| The microorganism identified under I above was accompanied by:<br><br>[ ] a scientific description<br><br>[ ] a proposed taxonomic designation<br><br>(Mark with a cross where applicable) |

| III. RECEIPT AND ACCEPTANCE |
|---|
| This International Depositary Authority accepts the microorganism identified under I above, which was received by it on **August 26, 2020.** |

| IV. RECEIPT OF REQUEST FOR CONVERSION |
|---|
| The microorganism identified under I above was received by this International Depositary Authority on and a request to convert the original deposit to a deposit under the Budapest Treaty was received by it on |

| V. INTERNATIONAL DEPOSITARY AUTHORITY | |
|---|---|
| Name: **Korean Collection for Type Cultures**<br><br>Address: Korea Research Institute of<br>Bioscience and Biotechnology (KRIBB)<br>181, Ipsin-gil, Jeongeup-si, Jeollabuk-do 56212<br>Republic of Korea | Signature(s) of person(s) having the power to represent the International Depositary Authority or of authorized official(s):<br><br>*Song-Gun Kim*<br><br>KIM, Song-Gun, Director<br>Date: **September 9, 2020** |

Form BP/4 (KCTC Form 17)      sole page

(Reissue)

# EP 4 206 317 A1

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

## RECEIPT IN THE CASE OF AN ORIGINAL DEPOSIT

issued pursuant to Rule 7.1

TO: Medytox Inc.

Medytox Inc.

114, Central town-ro, Yeongtong-gu, Suwon-si, Gyeonggi-do

Republic of Korea

| I. IDENTIFICATION OF THE MICROORGANISM | |
|---|---|
| Identification reference given by the DEPOSITOR: <br><br> *Enterococcus faecium* **LMT17-25** | Accession number given by the INTERNATIONAL DEPOSITARY AUTHORITY: <br><br> **KCTC 14288BP** |

### II. SCIENTIFIC DESCRIPTION AND/OR PROPOSED TAXONOMIC DESIGNATION

The microorganism identified under I above was accompanied by:

[ ] a scientific description

[ ] a proposed taxonomic designation

(Mark with a cross where applicable)

### III. RECEIPT AND ACCEPTANCE

This International Depositary Authority accepts the microorganism identified under I above, which was received by it on **August 26, 2020.**

### IV. RECEIPT OF REQUEST FOR CONVERSION

The microorganism identified under I above was received by this International Depositary Authority on and a request to convert the original deposit to a deposit under the Budapest Treaty was received by it on

### V. INTERNATIONAL DEPOSITARY AUTHORITY

| Name: **Korean Collection for Type Cultures** <br><br> Address: Korea Research Institute of Bioscience and Biotechnology (KRIBB) 181, Ipsin-gil, Jeongeup-si, Jeollabuk-do 56212 Republic of Korea | Signature(s) of person(s) having the power to represent the International Depositary Authority or of authorized official(s): <br><br> *Song-Gun Kim* <br><br> KIM, Song-Gun, Director <br> Date: **September 9, 2020** |
|---|---|

Form BP/4 (KCTC Form 17)                                        sole page.

(Reissue)

# EP 4 206 317 A1

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

## RECEIPT IN THE CASE OF AN ORIGINAL DEPOSIT

issued pursuant to Rule 7.1

TO: Medytox Inc.

Medytox Inc.

114, Central town-ro, Yeongtong-gu, Suwon-si, Gyeonggi-do

Republic of Korea

| I. IDENTIFICATION OF THE MICROORGANISM | |
|---|---|
| Identification reference given by the DEPOSITOR:<br><br>***Enterococcus faecium* LMT15-24** | Accession number given by the INTERNATIONAL DEPOSITARY AUTHORITY:<br><br>**KCTC 14289BP** |

II. SCIENTIFIC DESCRIPTION AND/OR PROPOSED TAXONOMIC DESIGNATION

The microorganism identified under I above was accompanied by:

[ ] a scientific description

[ ] a proposed taxonomic designation

(Mark with a cross where applicable)

III. RECEIPT AND ACCEPTANCE

This International Depositary Authority accepts the microorganism identified under I above, which was received by it on **August 26, 2020.**

IV. RECEIPT OF REQUEST FOR CONVERSION

The microorganism identified under I above was received by this International Depositary Authority on and a request to convert the original deposit to a deposit under the Budapest Treaty was received by it on

V. INTERNATIONAL DEPOSITARY AUTHORITY

| | Signature(s) of person(s) having the power to represent the International Depositary Authority or of authorized official(s): |
|---|---|
| Name: **Korean Collection for Type Cultures**<br><br>Address: Korea Research Institute of<br>Bioscience and Biotechnology (KRIBB)<br>181, Ipsin-gil, Jeongeup-si, Jeollabuk-do 56212<br>Republic of Korea | *Song-Gun Kim*<br><br>KIM, Song-Gun, Director<br>Date: **September 9, 2020** |

Form BP/4 (KCTC Form 17)                                                                 sole page

(Reissue)

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

## RECEIPT IN THE CASE OF AN ORIGINAL DEPOSIT

issued pursuant to Rule 7.1

TO: Medytox Inc.

Medytox Inc.

114, Central town-ro, Yeongtong-gu, Suwon-si, Gyeonggi-do

Republic of Korea

| I. IDENTIFICATION OF THE MICROORGANISM | |
|---|---|
| Identification reference given by the DEPOSITOR:<br><br>*Enterococcus faecium* **LMT15-4** | Accession number given by the INTERNATIONAL DEPOSITARY AUTHORITY:<br><br>**KCTC 14290BP** |

| II. SCIENTIFIC DESCRIPTION AND/OR PROPOSED TAXONOMIC DESIGNATION |
|---|
| The microorganism identified under I above was accompanied by:<br><br>[ ] a scientific description<br><br>[ ] a proposed taxonomic designation<br><br>(Mark with a cross where applicable) |

| III. RECEIPT AND ACCEPTANCE |
|---|
| This International Depositary Authority accepts the microorganism identified under I above, which was received by it on **August 26, 2020.** |

| IV. RECEIPT OF REQUEST FOR CONVERSION |
|---|
| The microorganism identified under I above was received by this International Depositary Authority on and a request to convert the original deposit to a deposit under the Budapest Treaty was received by it on |

| V. INTERNATIONAL DEPOSITARY AUTHORITY | |
|---|---|
| Name: **Korean Collection for Type Cultures**<br><br>Address: Korea Research Institute of<br>Bioscience and Biotechnology (KRIBB)<br>181, Ipsin-gil, Jeongeup-si, Jeollabuk-do 56212<br>Republic of Korea | Signature(s) of person(s) having the power to represent the International Depositary Authority or of authorized official(s):<br><br>*Song-Gun Kim*<br><br>KIM, Song-Gun, Director<br>Date: **September 9, 2020** |

Form BP/4 (KCTC Form 17)    sole page

(Reissue)

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

## RECEIPT IN THE CASE OF AN ORIGINAL DEPOSIT

issued pursuant to Rule 7.1

TO: Medytox Inc.

Medytox Inc.

114, Central town-ro, Yeongtong-gu, Suwon-si, Gyeonggi-do

Republic of Korea

| I. IDENTIFICATION OF THE MICROORGANISM | |
|---|---|
| Identification reference given by the DEPOSITOR:<br><br>*Enterococcus faecium* **LMT2-17** | Accession number given by the INTERNATIONAL DEPOSITARY AUTHORITY:<br><br>**KCTC 14291BP** |

**II. SCIENTIFIC DESCRIPTION AND/OR PROPOSED TAXONOMIC DESIGNATION**

The microorganism identified under I above was accompanied by:

[ ] a scientific description

[ ] a proposed taxonomic designation

(Mark with a cross where applicable)

**III. RECEIPT AND ACCEPTANCE**

This International Depositary Authority accepts the microorganism identified under I above, which was received by it on **August 26, 2020.**

**IV. RECEIPT OF REQUEST FOR CONVERSION**

The microorganism identified under I above was received by this International Depositary Authority on and a request to convert the original deposit to a deposit under the Budapest Treaty was received by it on

**V. INTERNATIONAL DEPOSITARY AUTHORITY**

| | |
|---|---|
| Name: **Korean Collection for Type Cultures**<br><br>Address: Korea Research Institute of Bioscience and Biotechnology (KRIBB)<br>181, Ipsin-gil, Jeongeup-si, Jeollabuk-do 56212<br>Republic of Korea | Signature(s) of person(s) having the power to represent the International Depositary Authority or of authorized official(s):<br><br>*Song-Gun Kim*<br><br>KIM, Song-Gun, Director<br>Date: **September 9, 2020** |

Form BP/4 (KCTC Form 17)  sole page.

(Reissue)

## EP 4 206 317 A1

## RECEIPT IN THE CASE OF AN ORIGINAL DEPOSIT

issued pursuant to Rule 7.1

TO: **Medytox Inc.**

Medytox Inc.

114, Central town-ro, Yeongtong-gu, Suwon-si, Gyeonggi-do

Republic of Korea

| I. IDENTIFICATION OF THE MICROORGANISM | |
|---|---|
| Identification reference given by the DEPOSITOR: <br><br> ***Enterococcus faecalis* LMT19-32** | Accession number given by the INTERNATIONAL DEPOSITARY AUTHORITY: <br><br> **KCTC 14306BP** |

| II. SCIENTIFIC DESCRIPTION AND/OR PROPOSED TAXONOMIC DESIGNATION |
|---|
| The microorganism identified under I above was accompanied by: <br><br> [ ] a scientific description <br><br> [ ] a proposed taxonomic designation <br><br> (Mark with a cross where applicable) |

| III. RECEIPT AND ACCEPTANCE |
|---|
| This International Depositary Authority accepts the microorganism identified under I above, which was received by it on **September 9, 2020.** |

| IV. RECEIPT OF REQUEST FOR CONVERSION |
|---|
| The microorganism identified under I above was received by this International Depositary Authority on and a request to convert the original deposit to a deposit under the Budapest Treaty was received by it on |

| V. INTERNATIONAL DEPOSITARY AUTHORITY | |
|---|---|
| Name: **Korean Collection for Type Cultures** <br><br> Address: Korea Research Institute of Bioscience and Biotechnology (KRIBB) 181, Ipsin-gil, Jeongeup-si, Jeollabuk-do 56212 Republic of Korea | Signature(s) of person(s) having the power to represent the International Depositary Authority or of authorized official(s): <br><br> *Song-Gun Kim* <br><br> KIM, Song-Gun, Director <br> Date: **September 9, 2020** |

Form BP/4 (KCTC Form 17)                                                      sole page

## Claims

1. A bacterial strain that

   i) belongs to a species *Enterococcus faecium;*
   ii) has a lactate production ability compared to microbial growth (lactate/$OD_{600}$) of 3 g/L or more, when cultured for 48 hours; and
   iii) exhibits anti-tumor activity.

2. The bacterial strain of claim 1, wherein the lactate production ability (lactate/$OD_{600}$) compared to microbial growth is greater than 2.5 g/L, when the bacterial strain is cultured for 24 hours.

3. The bacterial strain of claim 1 or 2, exhibiting a tumor inhibition rate of 10 % or more.

4. The bacterial strain of any one of claims 1 to 3, exhibiting a tumor inhibition rate of 20 % or more.

5. The bacterial strain of any one of claims 1 to 4, having iv) β-galactosidase activity.

6. The bacterial strain of any one of claims 1 to 5, having v) a D-sorbitol decomposition ability.

7. The bacterial strain of any one of claims 1 to 6, having vi) a D-tagatose decomposition ability.

8. The bacterial strain of any one of claims 1 to 7, having vii) a methyl-αD-mannopyranoside decomposition ability.

9. The bacterial strain of claim 1 or 2, which is at least one selected from the following groups:

   LMT17-62 deposited under an accession number of KCTC 14284BP;
   LMT17-74 deposited under an accession number of KCTC14285BP;
   LMT15-24 deposited under an accession number of KCTC 14289BP;
   LMT17-25 deposited under an accession number of KCTC 14288BP; and
   LMT15-4 deposited under an accession number of KCTC 14290BP.

10. A composition for preventing or treating tumor comprising a bacterial strain according to any one of claims 1 to 9, as an active ingredient.

11. The composition of claim 10 for oral administration.

12. The composition of claim 10 or 11, wherein the bacterial strain is live cells or dead cells.

13. The composition of any one of claims 10 to 12, wherein the tumor is solid tumor.

14. The composition of any one of claims 10 to 13, comprising the bacterial strain in an amount of $1 \times 10^6$ CFU or more.

15. The composition of any one of claims 10 to 14, for co-administrating with at least one other therapeutic agent.

16. The composition of claim 15, wherein the therapeutic agent is an immuno-anti-cancer agent.

17. The composition of claim 16, wherein immuno-anti-cancer agent is an immune checkpoint inhibitor.

18. The composition of claim 17, wherein the immune checkpoint inhibitor is a PD-1 antagonist, a PD-L1 antagonist, a CTLA-4 antagonist, or a combination thereof.

19. The composition of claim 18, wherein the immune checkpoint inhibitor is at least one selected from anti-PD-1 antibodies, anti-PD-L1 antibodies, anti-CTLA4 antibodies, or antigen-binding fragments thereof.

20. The composition of any one of claims 14 to 19, for administrating with at least one other therapeutic agent concurrently or sequentially.

21. A method of preventing or treating tumor in a subject comprising: a bacterial strain according to any one of claims 1 to 9, or a composition according to any one of claims 10 to 20, to a subject in need of prevention or treatment of tumor.

# FIG. 1

ENTEROCOCCUS FAECIUM
LMT17-62

ENTEROCOCCUS FAECIUM
KCTC13225

# FIG. 2A

FIG. 2B

DAYS AFTER TRANSPLANTION OF TUMOR CELLS

EP 4 206 317 A1

# FIG. 2C

# FIG. 3A

FIG. 3B

# FIG. 3C

# FIG. 4

# FIG. 5

# FIG. 6

# FIG. 7

ENTEROCOCCUS FAECALIS
LMT19-32

ENTEROCOCCUS FAECALIS
KCTC3206

# FIG. 8A

FIG. 8B

# FIG. 9A

# FIG. 9B

# FIG. 9C

## INTERNATIONAL SEARCH REPORT

| International application No. |
| --- |
| **PCT/KR2021/010525** |

| **A.** | **CLASSIFICATION OF SUBJECT MATTER** |
| --- | --- |

**C12N 1/20**(2006.01)i; **A61K 35/744**(2014.01)i; **A61P 35/00**(2006.01)i; **A23L 33/135**(2016.01)i; **C12P 7/56**(2006.01)i; **A61K 35/74**(2006.01)i; C12R 1/46(2006.01)n; **A61K 39/00**(2006.01)i

According to International Patent Classification (IPC) or to both national classification and IPC

| **B.** | **FIELDS SEARCHED** |
| --- | --- |

Minimum documentation searched (classification system followed by classification symbols)

C12N 1/20(2006.01); A23L 33/135(2016.01); A61K 31/11(2006.01); A61K 47/00(2006.01); A61K 47/26(2006.01); A61P 37/06(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 엔테로코커스 패시움(enterococcus faecium), 배양(culture), 락테이트(lactate), 생성능(formation potential), 항종양(anti-tumor), 항암(anti-cancer), 균주(strain)

| **C.** | **DOCUMENTS CONSIDERED TO BE RELEVANT** |
| --- | --- |

| Category* | Citation of document, with indication, where appropriate, of the relevant passages | Relevant to claim No. |
| --- | --- | --- |
| A | KR 10-2018-0081509 A (4D PHARMA RESEARCH LIMITED) 16 July 2018 (2018-07-16)<br>    See claims 1-2 and 6. | 1-3,9 |
| A | SIVIERI, K. et al. Probiotic Enterococcus faecium CRL 183 inhibit chemically induced colon cancer in male wistar rats. European Food Research and Technology. 2008, vol. 228, pp. 231-237.<br>    See entire document. | 1-3,9 |
| A | DE LA CRUZ-LOPEZ, K. G. et al. Latate in the regulation of tumor microenvironment and therapeutic approaches. Frontiers in Oncology. 2019, vol. 9, thesis no. : 1143 (pp. 1-21).<br>    See entire document. | 1-3,9 |
| A | WO 2020-079021 A1 (PHARMABIOME AG) 23 April 2020 (2020-04-23)<br>    See entire document. | 1-3,9 |
| A | WO 2020-079024 A1 (PHARMABIOME AG et al.) 23 April 2020 (2020-04-23)<br>    See entire document. | 1-3,9 |

| ☐ Further documents are listed in the continuation of Box C. | ☑ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **16 November 2021** | **16 November 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2021/010525**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**

International application No.

**PCT/KR2021/010525**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **21**
   because they relate to subject matter not required to be searched by this Authority, namely:

   Claim 21 pertains to a method for treatment of the human body by surgery or therapy, as well as a diagnostic method (PCT Article 17(2)(a)(i) and PCT Rule 39.1(iv)).

2. ☑ Claims Nos.: **11, 16-19**
   because they relate to parts of the international application that do not comply with the prescribed requirements to such an extent that no meaningful international search can be carried out, specifically:

   Since claims 11 and 16-19 refer to multiple dependent claims not meeting the requirement of PCT Rule 6.4(a), claims 11 and 16-19 are unclear.

3. ☑ Claims Nos.: **4-8, 10, 12-15, 20-21**
   because they are dependent claims and are not drafted in accordance with the second and third sentences of Rule 6.4(a).

Form PCT/ISA/210 (continuation of first sheet) (July 2019)

INTERNATIONAL SEARCH REPORT
Information on patent family members

International application No.

**PCT/KR2021/010525**

| Patent document cited in search report | | | Publication date (day/month/year) | Patent family member(s) | | | Publication date (day/month/year) |
|---|---|---|---|---|---|---|---|
| KR | 10-2018-0081509 | A | 16 July 2018 | CN | 108513544 | A | 07 September 2018 |
| | | | | EP | 3377082 | A1 | 26 September 2018 |
| | | | | JP | 2018-537397 | A | 20 December 2018 |
| | | | | JP | 2021-155458 | A | 07 October 2021 |
| | | | | JP | 6909152 | B2 | 28 July 2021 |
| | | | | US | 10046015 | B2 | 14 August 2018 |
| | | | | US | 10471108 | B2 | 12 November 2019 |
| | | | | US | 2017-0143773 | A1 | 25 May 2017 |
| | | | | US | 2019-0000892 | A1 | 03 January 2019 |
| | | | | US | 2020-0171098 | A1 | 04 June 2020 |
| | | | | WO | 2017-085518 | A1 | 26 May 2017 |
| WO | 2020-079021 | A1 | 23 April 2020 | | None | | |
| WO | 2020-079024 | A1 | 23 April 2020 | EP | 3866821 | A1 | 25 August 2021 |

Form PCT/ISA/210 (patent family annex) (July 2019)

**EP 4 206 317 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016196605 A
- WO 2017085520 A
- WO 2017085518 A
- KR 1020200054589
- KR 102053730